# EUROPEAN PATENT APPLICATION

(11) **EP 4 781 982 A1**
(43) Date of publication of application: **29.07.2026**
(21) Application number: 25222009.0
(22) Date of filing: 09.12.2025
(51) Int. Cl.: A61K 31/09, A61K 35/00, A61P 35/00

(54) **ANTI-CANCER AGENT CONTAINING 1,1-DIETHOXYETHANE AS AN ACTIVE INGREDIENT**

(30) Priority: 24.01.2025 KR 20250010954; 16.10.2025 KR 20250150102
(71) Applicant: Industry Foundation of Chonnam National University, Gwangju 61186 (KR); Lux Anima Co., Ltd., Jeollanam-do 58141 (KR)
(72) Inventor: LEE, Seung-Rock, Gwangju (KR); SAH, Dhiraj Kumar, Jeollanam-do (KR); CHOI, Jin Myung, Jeollanam-do (KR); LEE, Geunhaeng, Gwangju (KR); NGUYEN, Huu Thang, Jeollanam-do (KR); YOON, Hyun Joong, Gwangju (KR); TRINH, Hoang Vu, Jeollanam-do (KR)
(74) Representative: Vossius & Partner Patentanwälte Rechtsanwälte mbB

(57) **Abstract**

[Summary]

The present invention relates to an anticancer agent comprising 1,1-diethoxyethane (1,1-DEE) as an active ingredient.

## Description

### [Cross-Reference to Related Applications]

This application claims priority to Korean Patent Application Nos. 10-2025-0010954 filed on January 24, 2025, and 10-2025-0150102 filed on October 16, 2025, the entire contents of each of which are incorporated herein by reference.

### [Technical Field]

The present invention relates to an anti-cancer agent, and more particularly, to an anti-cancer agent comprising 1,1-diethoxyethane (1,1-diethoxyethane, 1,1-DEE) as an active ingredient.

### [Background Art]

Leukemia is one of the most common cancers in children and adolescents, accounting for approximately one-third of diagnosed cancer cases [1]. Acute lymphoblastic leukemia (ALL) is the most prevalent hematologic malignancy and is classified into B-cell precursor ALL (B-ALL) and T-cell precursor ALL (T-ALL) [2, 3]. T-cell acute lymphoblastic leukemia (T-ALL) is a rare form in children, representing approximately 12% to 15% of newly diagnosed cases and is distinguished by distinct clinical and molecular characteristics. Historically, the prognosis of T-ALL was poorer compared to B-ALL; however, advancements in treatment have improved outcomes, with event-free survival (EFS) reaching approximately 85% [4-6].

Leukemia can metastasize to various tissues, making its management complex [7]. Therefore, a better understanding of the complexity and mechanisms of antitumor immunity can contribute to the development of tumor immunotherapy and cancer biology [8]. In the development of anticancer agents, it is important to evaluate the specificity for inducing cancer cell death [9]. Approaches that induce the death of lymphoma cells represent unique therapeutic strategies, yet conventional apoptosis inducers may cause severe side effects due to their heavy metal content. Accordingly, it is essential to discover novel apoptosis inducers with higher efficacy and lower toxicity [10, 11]. Oxidative stress is a major mediator of lymphocyte apoptosis, and reactive oxygen species (ROS) are essential for various intracellular functions, particularly targeting polyunsaturated fatty acids abundant in actively proliferating lymphocytes. Excessive generation of ROS is suggested to promote cell death via mitochondrial damage, which activates signaling networks inducing cell cycle arrest, DNA repair, and apoptosis. Changes in cyclin expression play a critical role in regulating cell growth and malignant transformation.

### Prior Art References

[Non-Patent Document 1] Thimoteo, R.R.C., et al., Microarray data analysis of antileukemic action of Cinnamoylated benzaldehyde LQB-461 in Jurkat cell line. Mol Biol Rep, 2024. 51(1): p. 187.
[Non-Patent Document 2] Mirsanei, J.S., et al., Does Gold-Silver Core-Shell Nanostructure with Alginate Coating Induce Apoptosis in Human Lymphoblastic Tumoral (Jurkat) Cell Line Rep Biochem Mol Biol, 2023. 12(2): p. 233-240.
[Non-Patent Document 3] Caracciolo, D., et al., The emerging scenario of immunotherapy for T-cell Acute Lymphoblastic Leukemia: advances, challenges and future perspectives. Experimental Hematology & Oncology, 2023. 12(1): p. 5.
[Non-Patent Document 4] Raetz, E.A. and D.T. Teachey, T-cell acute lymphoblastic leukemia. Hematology Am Soc Hematol Educ Program, 2016. 2016(1): p. 580-588.
[Non-Patent Document 5] Zhong, F., et al., Hirsutanol A inhibits T-acute lymphocytic leukemia Jurkat cell viability through cell cycle arrest and p53-dependent induction of apoptosis. Exp Ther Med, 2021. 22(1): p. 741.
[Non-Patent Document 6] Sheykhhasan, M., H. Manoochehri, and P. Dama, Use of CAR T-cell for acute lymphoblastic leukemia (ALL) treatment: a review study. Cancer Gene Therapy, 2022. 29(8): p. 1080-1096.
[Non-Patent Document 7] Basaiyye, S.S., et al., Molecular mechanism of apoptosis induction in Jurkat E6-1 cells by Tribulus terrestris alkaloids extract. J Tradit Complement Med, 2018. 8(3): p. 410-419.
[Non-Patent Document 8] Rostami, F., et al., PDL1 targeting by miR-138-5p amplifies anti-tumor immunity and Jurkat cells survival in non-small cell lung cancer. Scientific Reports, 2024. 14(1): p. 13542.
[Non-Patent Document 9] Kumar, N., et al., Comparison of cell-based assays to quantify treatment effects of anticancer drugs identifies a new application for Bodipy-L-cystine to measure apoptosis. Scientific Reports, 2018. 8(1): p. 16363.
[Non-Patent Document 10] Zhu, X.L., et al., Inhibitory effect of Embelin on human acute T cell lymphoma Jurkat cells through activation of the apoptotic pathway. Oncol Lett, 2015. 10(2): p. 921-926.
[Non-Patent Document 11] Sun, Y.L., et al., A novel Bcl-2 inhibitor, BM-1197, induces apoptosis in malignant lymphoma cells through the endogenous apoptotic pathway. BMC Cancer, 2019. 20(1): p. 1.

### [Detailed Description of the Invention]

### [Technical Problem]

Abnormal cell proliferation is a hallmark of cancer, and cell cycle regulation is mediated by cyclins, particularly cyclin-dependent kinases (CDKs) that regulate the G1-S transition and G2 progression. Such cell cycle regulatory mechanisms play a critical role in cancer cell proliferation and survival, and therapeutic strategies targeting these mechanisms are required.

The inventors, as a result of continuous research to develop a compound exhibiting anticancer effects through inhibition of cancer cell growth and induction of apoptosis, confirmed that 1,1-diethoxyethane (1,1-DEE) shows a potent apoptotic effect in the T-lymphoblast leukemia cell line (Jurkat E6.1) (Example 1). Specifically, upon treatment with 1,1-DEE, morphological changes such as cell rounding and shrinkage were observed, and Annexin V/PI staining and Western blot analysis revealed an increase in Bax protein expression and a decrease in Bcl-2, caspase-3, and caspase-9 expression. In contrast, these effects were not observed in the group treated with 1,2-DEE, an isomer of 1,1-DEE.

In addition, 1,1-DEE was demonstrated to induce the generation of intracellular reactive oxygen species (ROS), thereby reducing the protein levels of CDK3, CDK4, cyclin D1, D3, and E and inducing cell cycle arrest at the G0/G1 phase (Example 2). Pretreatment with ROS scavengers (NAC, Trolox, Ebselen) inhibited apoptosis and cell cycle arrest, indicating that the anticancer activity of 1,1-DEE is associated with a ROS-mediated mechanism.

Furthermore, 1,1-DEE was found to activate AMPK signaling, regulate metabolic reprogramming in cancer cells, and inhibit the Warburg effect, thereby decreasing the expression of glycolytic enzymes (LDHA, HK2) and glucose transporter (GLUT1), while maintaining mitochondrial oxidative phosphorylation (OXPHOS) activity (Examples 3 and 4). This metabolic regulation, in conjunction with apoptosis and cell cycle arrest, demonstrates an inhibitory effect on cancer cell growth and survival.

In addition, in the NSG mouse model, 1,1-DEE was confirmed to suppress tumor growth, increase survival rate, minimize histological damage in major organs, and reduce leukemia-associated inflammatory biomarker (IL-8) levels (Example 5).

As described above, the present invention demonstrates that 1,1-DEE can effectively inhibit the growth and survival of cancer cells through ROS generation, cell cycle regulation, AMPK-mediated metabolic reprogramming, and induction of apoptosis, thereby providing a foundation for the development of anticancer compositions and methods for the prevention and treatment of cancer utilizing the same.

However, the problems to be solved by the present invention are not limited to those mentioned above, and other problems not specifically described herein will be clearly understood by those skilled in the art from the following description.

### [Technical Solution]

The present invention discloses an anticancer agent comprising 1,1-diethoxyethane (1,1-DEE) as an active ingredient.

In the present invention, 1,1-diethoxyethane (1,1-DEE) is represented by the molecular formula C₆H₁₄O₂ and the following structural formula 1, and is also referred to as acetaldehyde diethyl acetal or ethyllidene diethyl ether.

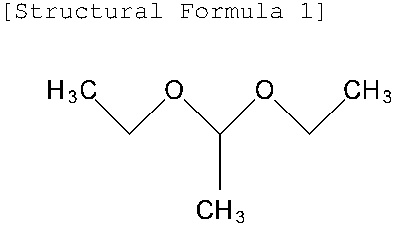

In the present invention, the 1,1-DEE may induce the generation of reactive oxygen species (ROS) in cancer cells.

In the present invention, the 1,1-DEE may induce apoptosis in cancer cells.

In the present invention, the 1,1-DEE may induce cell cycle arrest by depleting cells in the G2/M phase and accumulating cells in the G1 phase, and may reduce the expression of CDK3, CDK4, and cyclins D1, D3, and E.

In the present invention, the anticancer agent may be used for haematological malignancies or solid tumors.

In the present invention, the anticancer agent may be used for cancers selected from the group consisting of squamous cell carcinoma, basal cell carcinoma, melanoma, tumors of epithelial lining of glands or ducts, adenocarcinoma, papillary carcinoma, papillary adenocarcinoma of the liver and gallbladder ducts, hepatocellular carcinoma of the gastrointestinal tract, esophageal squamous cell carcinoma, esophageal adenocarcinoma, colorectal cancer, gastric cancer, airway tumors, bronchogenic carcinoma, small cell carcinoma, urinary tract transitional cell carcinoma, bladder squamous cell carcinoma, prostate cancer, cervical cancer, leukemia of hematopoietic cells and related cells, acute and chronic lymphocytic leukemia, polycythemia vera, lymphoid tissue cancers, Hodgkin lymphoma and non-Hodgkin lymphoma including malignant lymphomas, follicular lymphoma, diffuse lymphoma, small lymphocytic lymphoma, large cell lymphoma, lymphoblastic lymphoma, multiple myeloma, connective tissue tumors, osteosarcoma, nervous system tumors, neuroblastoma, retinoblastoma, glioblastoma, tumorigenic virus-associated glioma, Burkitt lymphoma, B cell lymphoma in immunocompetent subjects, nasopharyngeal carcinoma, esophageal and gastroesophageal cancer, squamous cell carcinoma, pancreatic islet tumors, breast cancer, lung cancer, colorectal cancer, retinoblastoma, liver cancer, pancreatic cancer, brain cancer, malignant mesothelioma, hepatitis B virus-related hepatocellular carcinoma, endometrial cancer, ovarian cancer, head and neck cancer, thyroid cancer, and soft tissue-related cancers.

In the present invention, when the anticancer agent is used for leukemia, the leukemia may be selected from the group consisting of acute lymphoblastic leukemia (ALL), acute lymphoblastic B-cell leukemia, acute lymphoblastic T-cell leukemia, acute myeloblastic leukemia (AML), acute promyelocytic leukemia (APL), acute monocytic leukemia, acute erythroid leukemia, acute megakaryoblastic leukemia, acute myelomonocytic leukemia, acute undifferentiated leukemia, chronic myeloid leukemia, chronic lymphocytic leukemia, T-cell precursor lymphoblastic leukemia, T-cell prolymphocytic leukemia, Philadelphia chromosome-positive leukemia, FLT3 mutation-positive acute myeloblastic leukemia, and acute erythroid leukemia.

In the present invention, the anticancer agent may be administered in combination with one or more of an immunotherapeutic agent, monoclonal antibody, chemotherapeutic agent, radioprotective agent, radiotherapeutic agent, and gene therapy agent. The immunotherapeutic agent may include immune checkpoint inhibitors comprising PD-1, PD-L1, and CTLA-4 inhibitors, cytokine therapy comprising interleukins and interferons, CAR-T cell therapy, oncolytic viruses, vaccine therapy, or targeted therapy; the chemotherapeutic agent may include antimetabolites, platinum-based compounds, alkylating agents, or topoisomerase inhibitors; and the radiotherapeutic agent may include external beam radiation, brachytherapy, proton therapy, radiofrequency ablation, stereotactic radiosurgery, or neutron therapy.

In the present invention, the anticancer agent may be used to prevent the onset of cancer.

In the present invention, the anticancer agent may exhibit an effect of restoring vitality during the course of cancer treatment.

In the present invention, the anticancer agent may be used as a pharmaceutical composition for preventing or treating cancer. The pharmaceutical composition may be administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, and rectal administration.

In the present invention, the anticancer agent may be used as a cosmetic composition for preventing or improving cancer. The cosmetic composition may be formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, and spray.

In the present invention, the anticancer agent may be used as a food composition for preventing or improving cancer. The food may include meat, sausage, bread, chocolate, candy, snacks, cookies, pizza, ramen, other noodles, chewing gum, ice cream and dairy products, various soups, beverages, tea, coffee beverages, stamina drinks, alcoholic beverages, or vitamin complexes.

In the present invention, the anticancer agent may be used as a feed composition for preventing or improving cancer. The feed may include powdered feed, solid feed, moist pellet feed, dry pellet feed, extruder pellet (EP) feed, or raw feed.

### [Effects of the Invention]

According to the present invention, 1,1-diethoxyethane (1,1-DEE) was shown to decrease cell viability in a concentration-dependent manner in the T-lymphoblast leukemia cell line (Jurkat E6.1), induce morphological changes, and promote apoptosis through increased Bax protein expression and decreased expression of Bcl-2, caspase-3, and caspase-9 (Example 1). In addition, 1,1-DEE was found to induce ROS generation, and these effects were confirmed to be suppressible by ROS scavengers (Example 2). Cell cycle analysis demonstrated that 1,1-DEE induces cell cycle arrest by depleting cells in the G2/M phase and accumulating cells in the G1 phase, and reduces the levels of CDK3, CDK4, and cyclins D1, D3, and E, thereby contributing to the inhibition of cancer cell proliferation (Example 2).

Furthermore, 1,1-DEE was shown to activate AMPK signaling, suppress the expression of glycolytic enzymes (LDHA, HK2) and glucose transporter (GLUT1), and inhibit the Warburg effect by maintaining or enhancing mitochondrial oxidative phosphorylation (OXPHOS) activity, thereby regulating metabolic reprogramming in cancer cells (Examples 3 and 5).

In in vivo experiments (NSG mice), 1,1-DEE significantly suppressed tumor growth and increased survival, while minimizing histological damage in major organs and reducing the expression of leukemia-associated inflammatory biomarker (IL-8), thereby demonstrating its potential as an anticancer agent (Example 4).

Therefore, the present invention provides a novel anticancer treatment method using 1,1-DEE, and is expected to contribute to the development of a new type of anticancer agent that, compared to conventional therapies, exhibits lower toxicity and higher efficacy, while promoting prevention, treatment, and recovery of cancer through AMPK-mediated regulation of energy metabolism.

Meanwhile, the scope of the present invention is not limited by the effects described above.

### [Brief Description of Drawings]

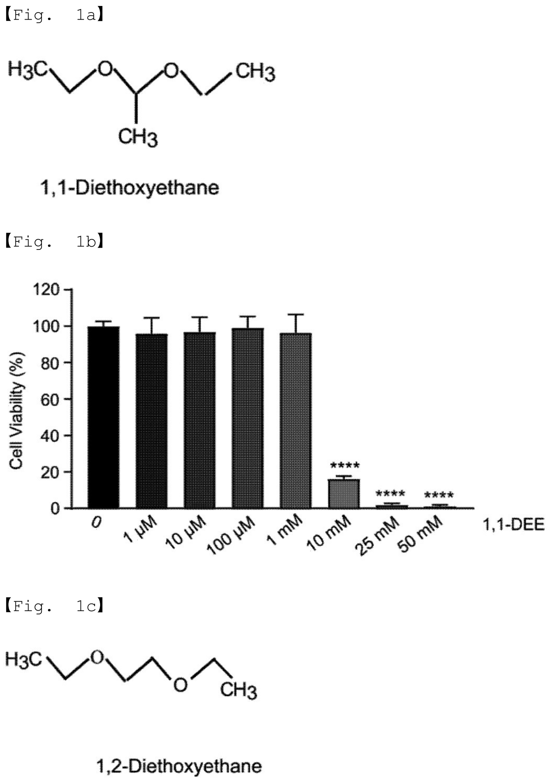

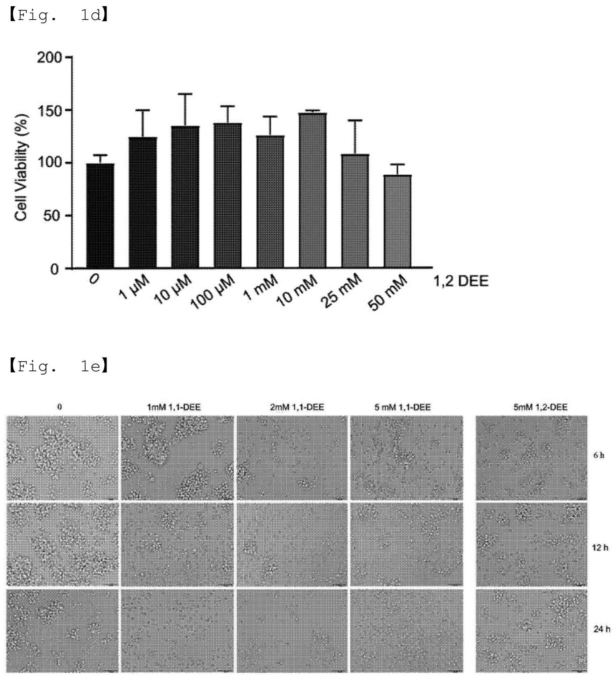

Figure 1 shows the results of cell viability assessment using MTT assay. Cells were treated with 1,1-DEE (A and B) and its structural isomer 1,2-DEE (C and D) at the indicated concentrations for 24 hours, followed by reaction with 10 µL of MTT solution for 2 hours. Each experiment was performed in triplicate, and a total of three independent experiments were conducted. Data are presented as mean ± standard error of the mean (SEM), with *p < 0.05 and ****p < 0.0001 indicating statistical significance compared to the control.

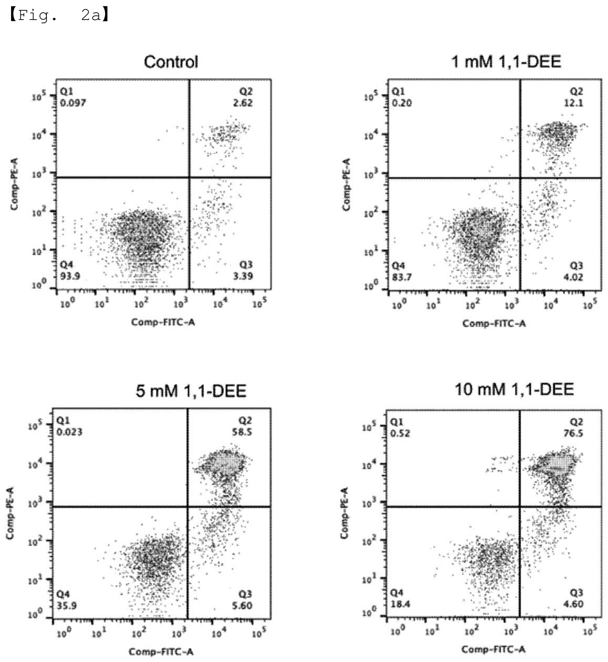

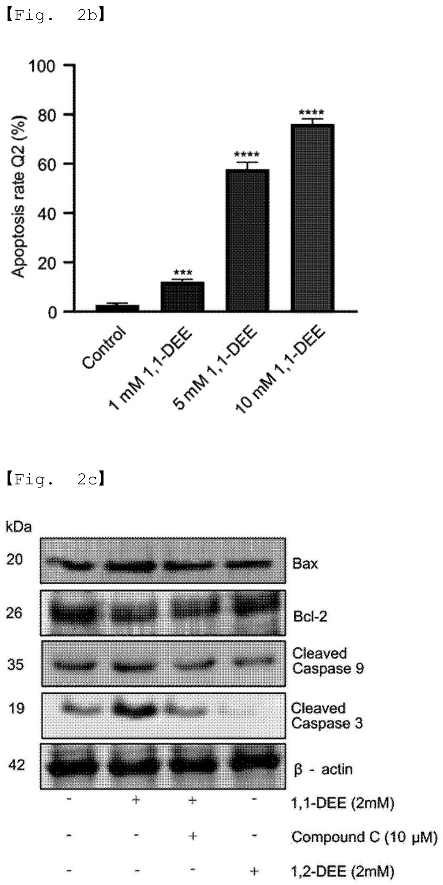

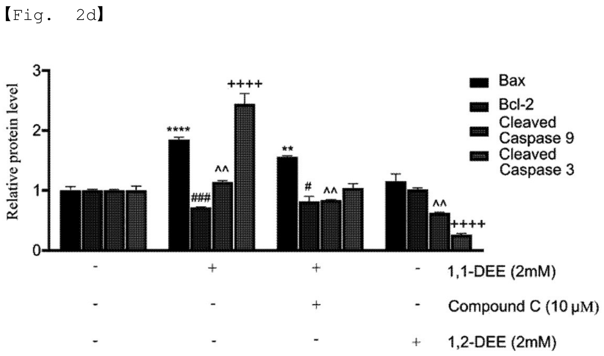

Figure 2 shows the analysis of apoptosis in Jurkat cells using flow cytometry. Treatment with 1,1-DEE at different concentrations for 24 hours resulted in a significant increase in the number of apoptotic cells compared to the control. (A) and (B) show the results of Annexin V and PI staining and the corresponding apoptotic cell percentages, while (C) and (D) present the Western blot analysis of pro-apoptotic and anti-apoptotic protein expression. Data are presented as mean ± standard deviation (SD) of three independent experiments, and statistical significance was confirmed at p < 0.0001. Statistical significance is indicated as *p < 0.0001 and ****p < 0.00001, compared to the control group.

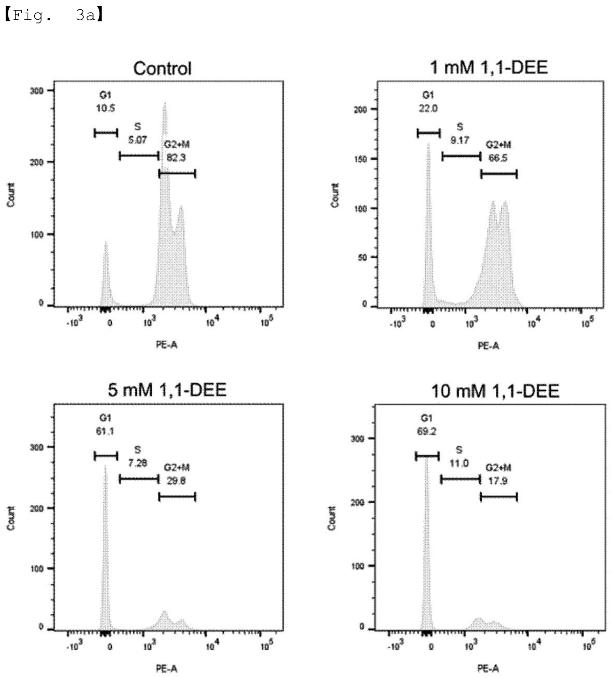

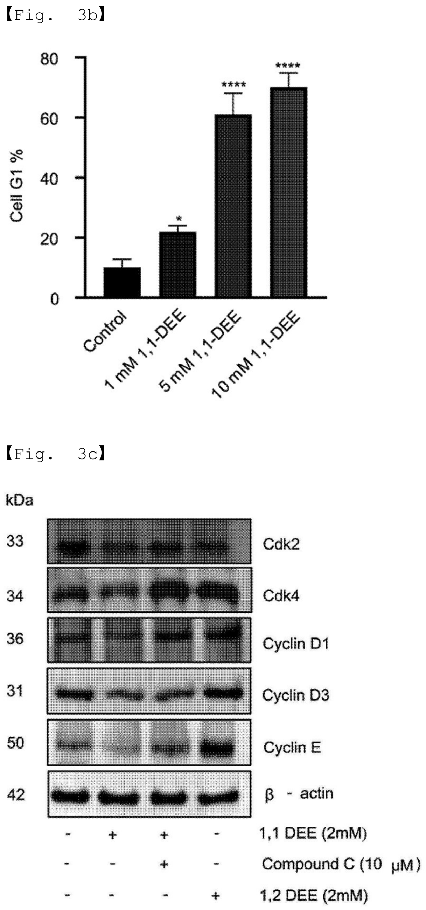

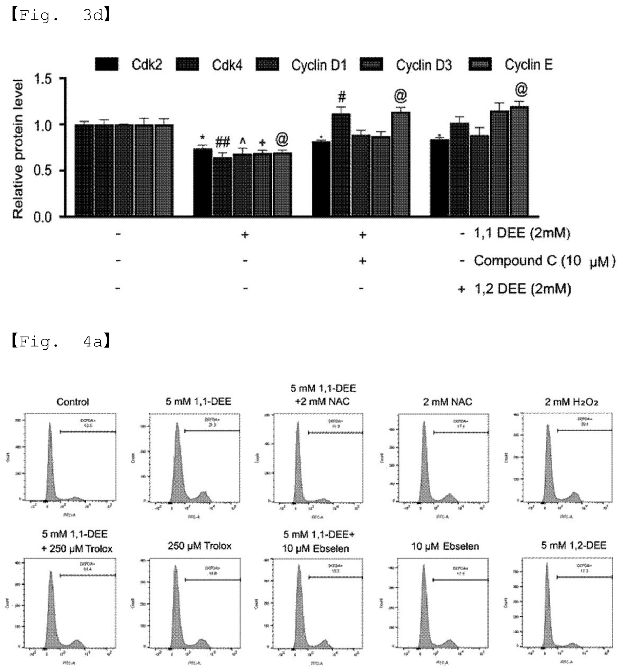

Figure 3 shows the results of cell cycle analysis of Jurkat cells after treatment with 1,1-DEE at 1-10 mM for 24 hours. (A) presents representative flow cytometry results for cell cycle arrest, and (B) shows the percentages of cells in S phase and G2/M phase, as well as the proportion of early apoptotic cells observed in the sub-G1 peak. (C) and (D) show the results of Western blot analysis. Data are presented as mean ± standard deviation (SD) of three independent experiments, and statistical significance is indicated by *p < 0.05, compared to the control.

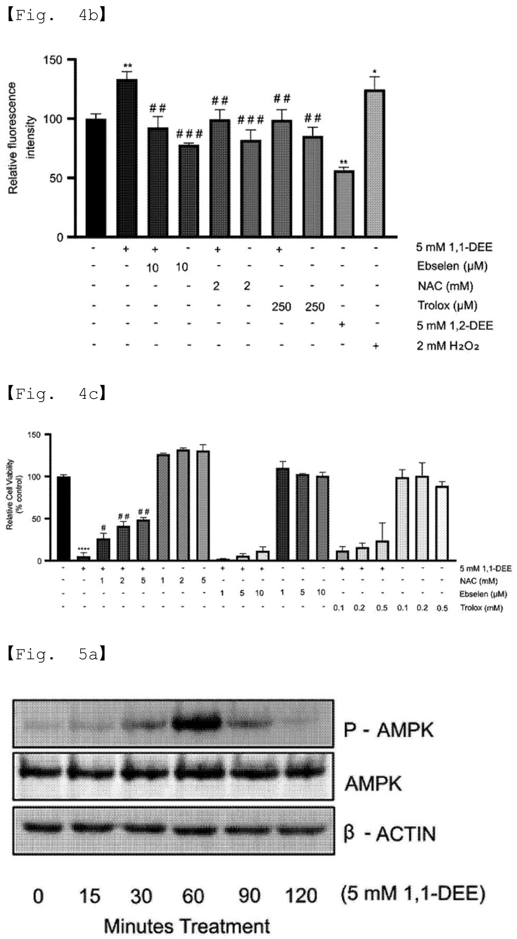

Figure 4 shows the results of ROS generation induced by 1,1-DEE treatment. (A) shows flow cytometry analysis of ROS generation in Jurkat cells pretreated with NAC (2 mM), Trolox (250 µM), or Ebselen (10 µM) followed by treatment with 1, 1-DEE (5 mM) or H₂O₂ (2 mM). (B) shows the ROS fluorescence intensity measured after treatment with 1,1-DEE or 1,2-DEE and/or ROS scavenger treatment. (C) shows the evaluation of cell viability changes mediated by ROS scavengers using MTT assay, where cells were pretreated for 1 hour with NAC (1-5 mM), Trolox (125-500 µM), or Ebselen (1-10 µM), followed by treatment with 5 mM 1,1-DEE for 24 hours. Statistical significance is indicated as *p < 0.05 compared to the control group and #p < 0.05 compared to the 1,1-DEE-treated group. Data are presented as mean ± SD of three independent experiments.

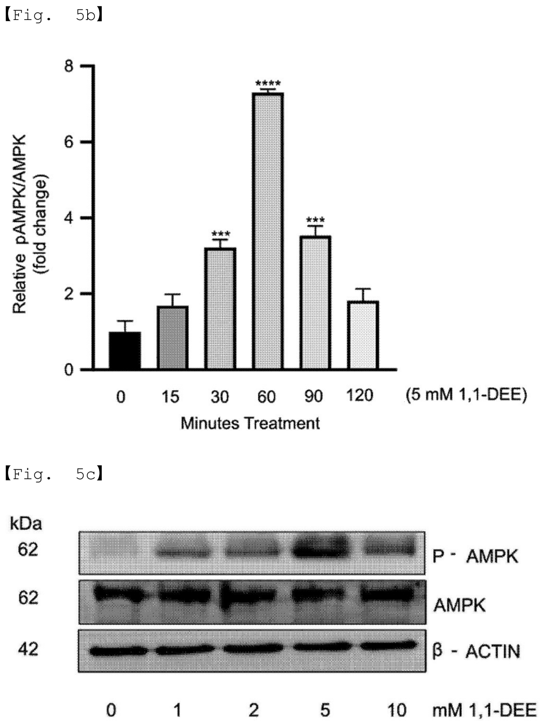

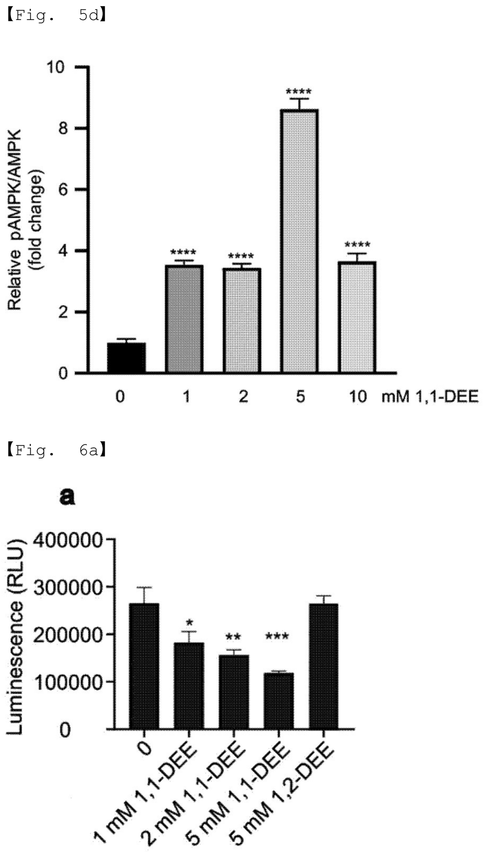

Figure 5 shows the results of AMPK activation in Jurkat cells upon 1,1-DEE treatment. Jurkat cells were treated with 1,1-DEE for 0-120 minutes, and total AMPK and phosphorylated AMPK (p-AMPK) levels were analyzed by Western blot. (A) shows the time-dependent activation, and (C) shows the concentration-dependent activation. (B) and (D) present the quantification of p-AMPK/AMPK from Western blot. Statistical significance is indicated as *p < 0.05 compared to the control, and data are presented as mean ± SD of three independent experiments.

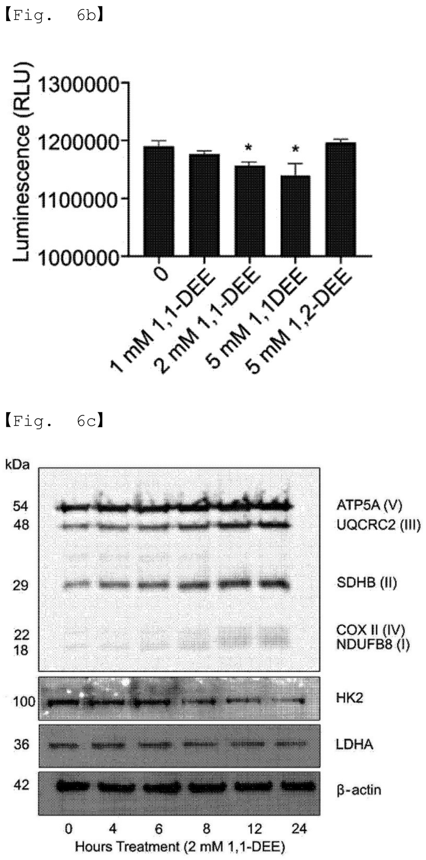

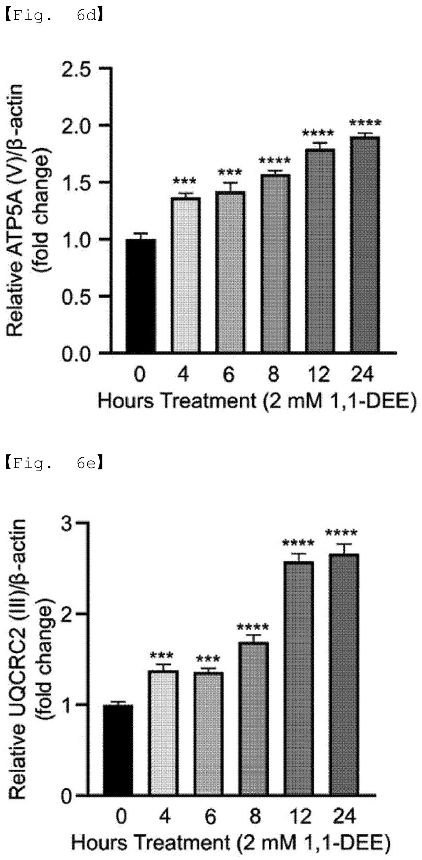

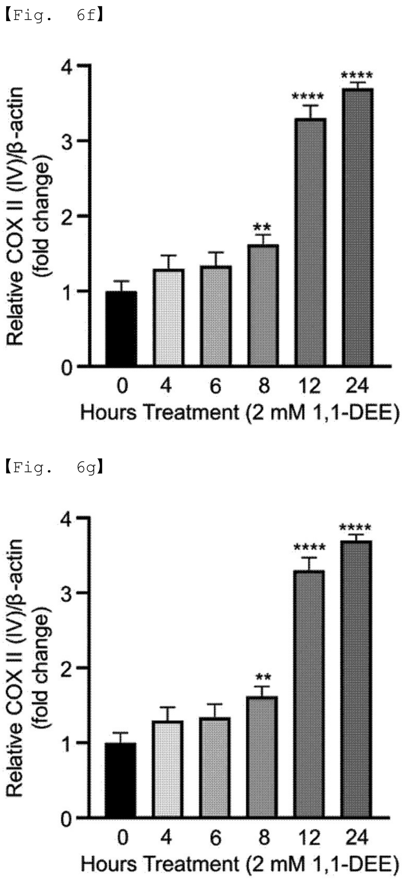

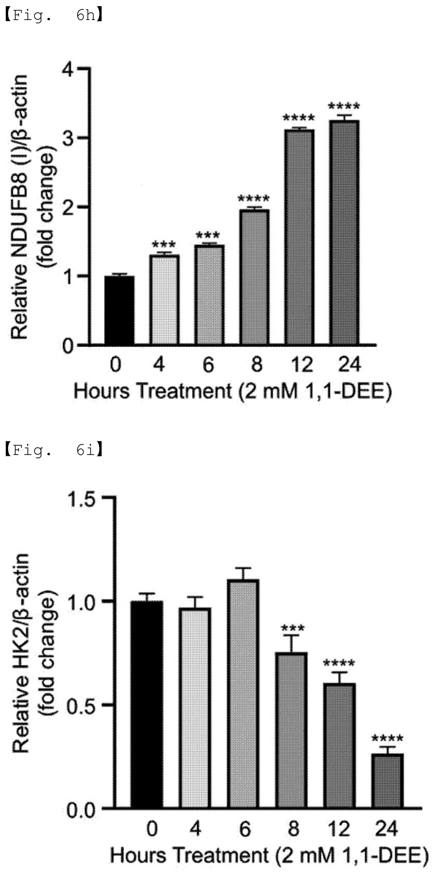

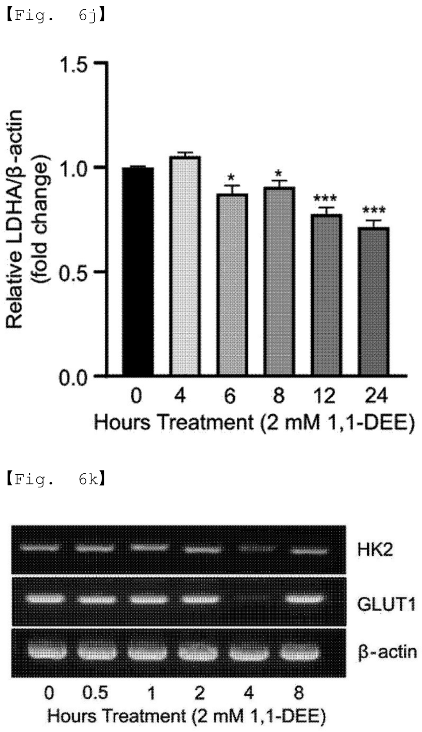

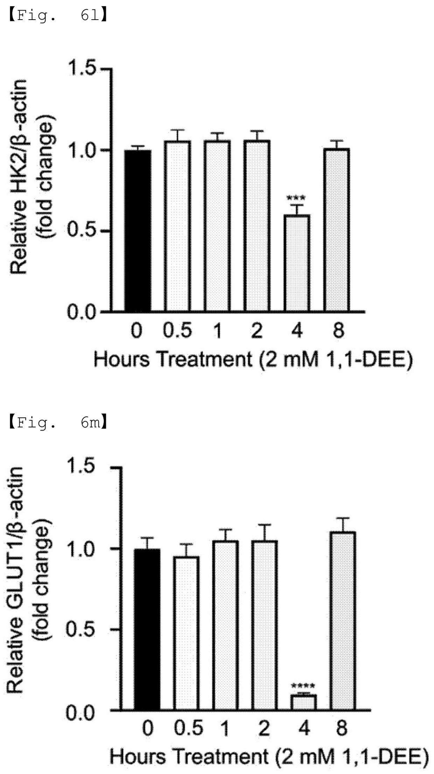

Figure 6 shows the effects of 1,1-DEE treatment on mitochondrial protein expression and glycolytic metabolism. Mitochondrial protein expression was evaluated using an antibody cocktail targeting five mitochondrial oxidative phosphorylation (OXPHOS) complex proteins. Jurkat cells were treated with 1-5 mM 1,1-DEE and, as a negative control, 5 mM 1,2-DEE for 2 hours, after which (A) glucose uptake and (B) lactate production were measured. Additionally, Jurkat cells were treated with 1,1-DEE for 2-24 hours. (C) shows representative Western blot results, and (D-H) show the quantification of OXPHOS complex proteins. (I-J) include glycolytic analysis, and the expression of glycolytic enzymes HK2 and LDHA was also evaluated after 1,1-DEE treatment. (K) shows time-dependent HK2 and GLUT1 expression analyzed by RT-PCR, and (L) and (M) show the quantification of the RT-PCR results. Data are presented as mean ± SEM of three independent experiments. Statistical significance is indicated as *p < 0.05 compared to the control and #p < 0.05 compared to 1,1-DEE.

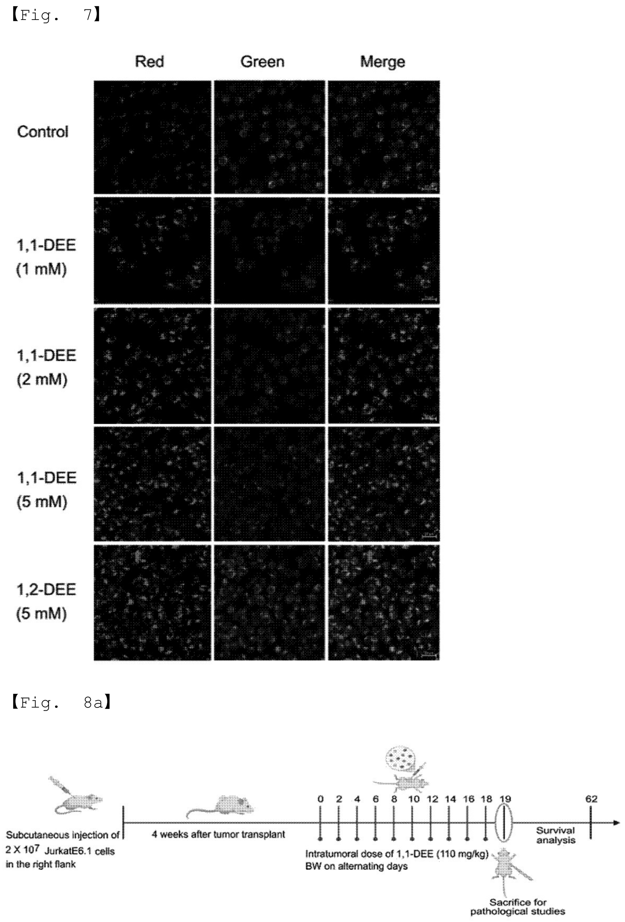

Figure 7 shows the effects of 1,1-DEE treatment on mitochondrial membrane potential in Jurkat cells. Jurkat cells were treated with 1-5 mM 1,1-DEE, and 1,2-DEE was used as a negative control. (A) presents representative confocal microscopy images (200× magnification) showing mitochondrial membrane potential polarization, assessed by JC-1 staining for 30 minutes after 1 hour of treatment.

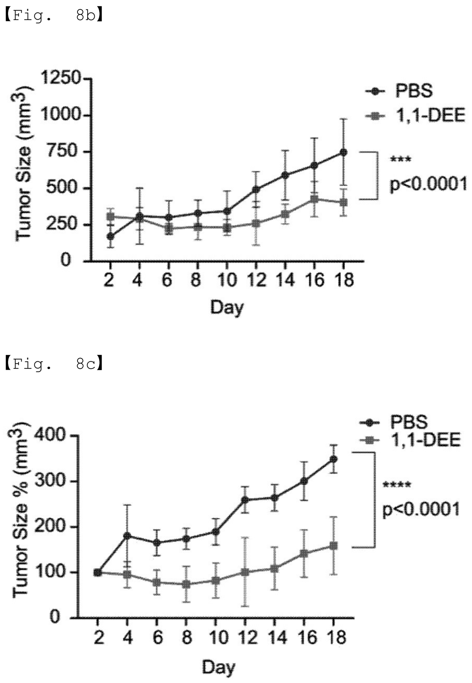

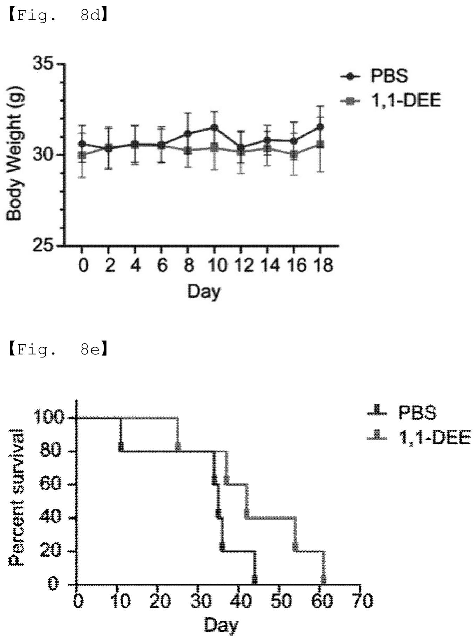

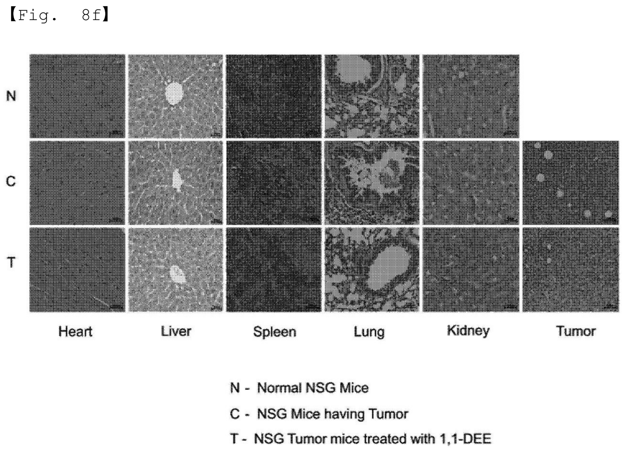

Figure 8 shows the results of Jurkat cell growth in a xenograft mouse model. (A) Jurkat cells (2 × 10⁷ cells) were unilaterally injected into the left leg of mice via subcutaneous injection into the depilated thigh, and tumor growth was monitored for 4 weeks. In the treatment group, 1,1-DEE (110 mg/kg) was administered by intratumoral injection on days 2, 4, 6, 8, 10, 12, 14, and 16, while the control group received PBS. The collected data include: (B) tumor volume in treatment and control groups, (C) tumor volume growth rate, (D) mouse body weight, (E) Kaplan-Meier survival curve of tumor-bearing mice, and (F) H&E staining of heart, liver, spleen, lung, and kidney. A significant difference in survival curves between the PBS control and 1,1-DEE treatment groups was observed, with p < 0.05, indicating statistical significance compared to PBS.

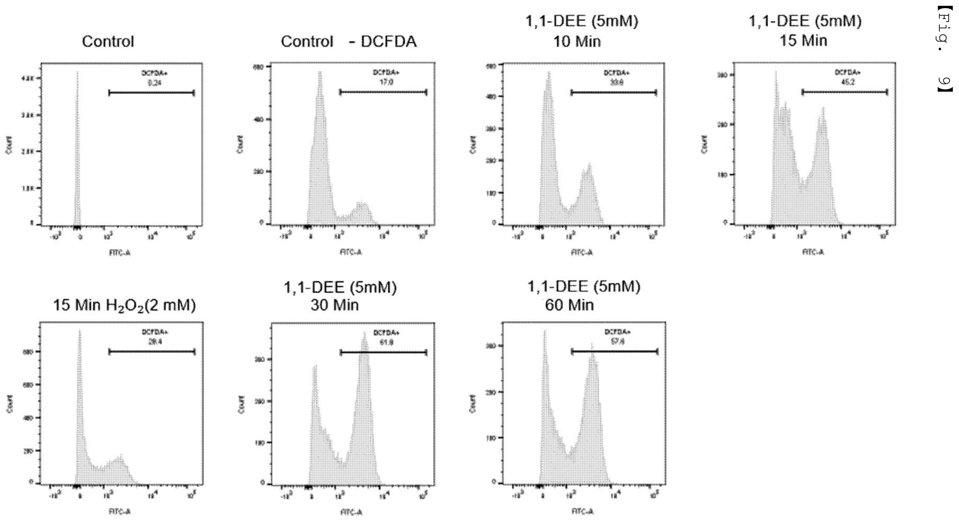

Figure 9 shows ROS levels measured at different time intervals using DCFDA staining. Representative flow cytometry histograms demonstrate the time-dependent accumulation of ROS in Jurkat cells treated with 5 mM 1,1-DEE for varying durations.

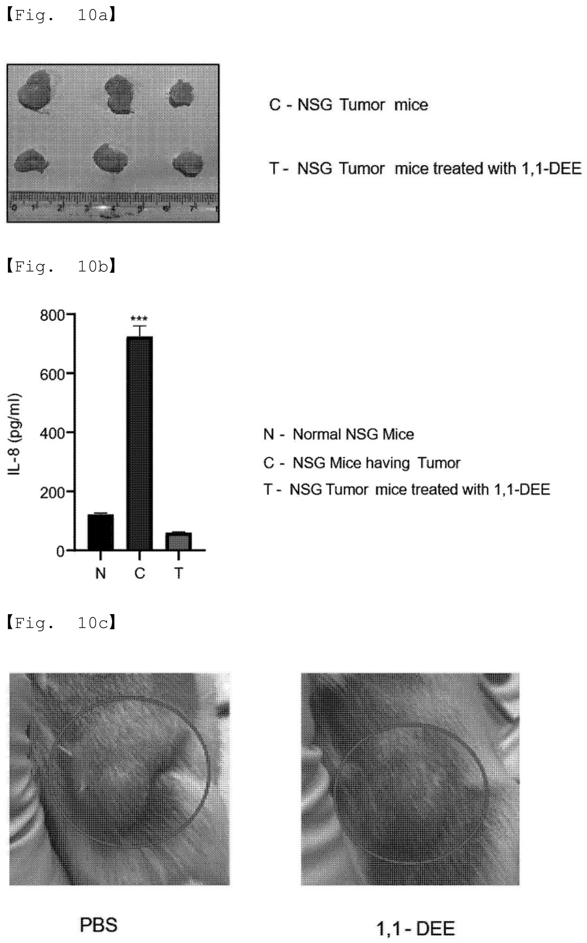

Figure 10 shows the effects of 1,1-DEE on tumor growth and inflammatory cytokine levels in mice. (A) Comparison of tumor size between the control (PBS) and 1,1-DEE-treated groups, (B) Levels of the inflammatory cytokine IL-8 measured in normal mice, untreated tumor-bearing mice, and 1,1-DEE-treated tumor-bearing mice, (C) Representative tumor images from PBS- and 1,1-DEE-treated mice. Data are presented as mean ± SEM of three independent experiments, and statistical significance is indicated as ***p < 0.0001 compared to the normal group (N).

### [DETAILED DESCRIPTION OF THE EXEMPLARY EMBODIMENTS]

Hereinafter, the present invention will be described in detail with respect to an anticancer agent comprising 1,1-diethoxyethane (1,1-DEE) as an active ingredient, according to specific embodiments of the invention. However, this is presented merely as one example of the invention, and the scope of the invention is not limited thereby. It will be apparent to those skilled in the art that various modifications of the embodiments are possible within the scope of the claims of the invention. Unless otherwise specifically stated in the present specification, the terms "comprising" or "containing" refer to the inclusion of certain components (or constituents) without particular limitation, and should not be construed as excluding the presence of other components (or constituents).

As used herein, the term "treatment" refers to any form of therapy or prevention administered to a subject who has a disease or is at risk of developing a disease, and provides effects such as improvement of the subject's condition, delay in disease progression, delay in symptom onset, or slowing of symptom progression. Accordingly, the term "treatment" encompasses prophylactic treatment to prevent the occurrence of symptoms. In addition, the terms "treatment" and "prevention" are not intended to imply the complete cure or elimination of symptoms.

As used herein, the term "improvement" may refer to any action that alleviates a condition or reduces at least the severity of symptoms or parameters related to treatment.

As used herein, the term "subject" refers to an animal, including, but not limited to, cattle, monkeys, horses, sheep, pigs, chickens, turkeys, quails, cats, dogs, mice, rats, rabbits, or guinea pigs. For example, the subject may be a mammal, and particularly, a human.

### 1. Anticancer Agent

The present invention provides an anticancer agent comprising 1,1-diethoxyethane (1,1-DEE) as an active ingredient.

The 1,1-DEE may induce the generation of reactive oxygen species (ROS) in cancer cells.

The 1,1-DEE may induce apoptosis in cancer cells.

The 1,1-DEE may induce cell cycle arrest in cancer cells through G2/M phase depletion and G1 phase accumulation, and can reduce the expression of CDK3, CDK4, and cyclin D1, D3, and E proteins.

The 1,1-DEE may activate AMPK signaling and suppress the expression of glycolytic enzymes (LDHA, HK2) and glucose transporter (GLUT1) in cancer cells.

The 1,1-DEE may maintain or enhance mitochondrial membrane potential (ΔΨm) and oxidative phosphorylation (OXPHOS) activity.

The 1,1-DEE may reduce the expression of leukemia-associated inflammatory biomarkers (IL-8).

The anticancer agent may be used for haematological malignancies or solid tumors.

The anticancer agent may be used for leukemia, multiple myeloma, or lymphoma. For example, the anticancer agent may be used for cancers selected from the group consisting of squamous cell carcinoma, basal cell carcinoma, melanoma, tumors of epithelial lining of glands or ducts, adenocarcinoma, papillary carcinoma, papillary adenocarcinoma of the liver and gallbladder ducts, hepatocellular carcinoma of the gastrointestinal tract, esophageal squamous cell carcinoma, esophageal adenocarcinoma, colorectal cancer, gastric cancer, airway tumors, bronchogenic carcinoma, small cell carcinoma, urinary tract transitional cell carcinoma, bladder squamous cell carcinoma, prostate cancer, cervical cancer, leukemia of hematopoietic cells and related cells, acute and chronic lymphocytic leukemia, polycythemia vera, lymphoid tissue cancers, Hodgkin lymphoma and non-Hodgkin lymphoma including malignant lymphomas, follicular lymphoma, diffuse lymphoma, small lymphocytic lymphoma, large cell lymphoma, lymphoblastic lymphoma, multiple myeloma, connective tissue tumors, osteosarcoma, nervous system tumors, neuroblastoma, retinoblastoma, glioblastoma, tumorigenic virus-associated glioma, Burkitt lymphoma, B cell lymphoma in immunocompetent subjects, nasopharyngeal carcinoma, esophageal and gastroesophageal cancer, squamous cell carcinoma, pancreatic islet tumors, breast cancer, lung cancer, colorectal cancer, retinoblastoma, liver cancer, pancreatic cancer, brain cancer, malignant mesothelioma, hepatitis B virus-related hepatocellular carcinoma, endometrial cancer, ovarian cancer, head and neck cancer, thyroid cancer, and soft tissue-related cancers.

When the anticancer agent may be used for leukemia, the leukemia may be selected from the group consisting of acute lymphoblastic leukemia (ALL), B-cell acute lymphoblastic leukemia, T-cell acute lymphoblastic leukemia, acute myeloblastic leukemia (AML), acute promyelocytic leukemia (APL), acute monoblastic leukemia, acute erythroid leukemia, acute megakaryoblastic leukemia, acute myelomonocytic leukemia, acute undifferentiated leukemia, chronic myeloid leukemia (CML), chronic lymphocytic leukemia (CLL), T-cell precursor lymphoblastic leukemia, T-cell prolymphocytic leukemia, Philadelphia chromosome-positive leukemia, FLT3-mutated acute myeloblastic leukemia, and acute erythroid leukemia.

The anticancer agent may be administered alone or in combination with one or more of an immunotherapeutic agent, a monoclonal antibody, a chemotherapeutic agent, a radioprotective agent, a radiotherapeutic agent, or a gene therapeutic agent such as microRNA. In this regard, the immunotherapeutic agent may include an immune checkpoint inhibitor such as a PD-1, PD-L1, or CTLA-4 inhibitor; a cytokine therapy comprising interleukins and interferons; CAR-T cell therapy; oncolytic viruses; a vaccine therapy; or a targeted therapy. The chemotherapeutic agent may include an antimetabolite, a platinum-based compound, an alkylating agent, or a topoisomerase inhibitor. The radiotherapeutic agent may include external beam radiation therapy, brachytherapy, proton therapy, radiofrequency ablation, stereotactic radiosurgery, or neutron therapy. In particular, the administration of the anticancer agent may, without being limited thereto, occur before, during, or after the administration of one or more known antineoplastic agents, including hypomethylating agents, PD-1 inhibitors, PD-L1 inhibitors, mustard compounds, nitrogen mustards, chlorambucil, melphalan, cyclophosphamide, 6-mercaptopurine, 6-thioguanine, cytarabine, 5-fluorouracil, floxuridine, methotrexate, vincristine, vinblastine, paclitaxel, etoposide, teniposide, dactinomycin, daunorubicin, doxorubicin, bleomycin, mitomycin, cisplatin, carboplatin, estramustine phosphate, hydroxyurea, BCNU, procarbazine, VM-26, interferon, and all-trans retinoic acid (ATRA), or other retinoids. Suitable hypomethylating agents may include decitabine, guadecitabine, and azacitidine. Suitable PD-1 inhibitors may include pembrolizumab and nivolumab, and suitable PD-L1 inhibitors may include atezolizumab, avelumab, and durvalumab.

In the anticancer agent according to the present invention, the anticancer agent may include a pharmaceutical composition, cosmetic composition, food composition, or feed composition.

### (1) Pharmaceutical Composition

According to the present invention, the anticancer agent may be used as a pharmaceutical composition for preventing or treating cancer.

In the pharmaceutical composition according to the present invention, the composition may be administered via oral administration, intravenous injection, subcutaneous injection, intramuscular injection, intraperitoneal injection, epithelial administration, topical administration, vaginal administration, pulmonary administration, rectal administration, sublingual administration, buccal administration, transdermal administration, ocular administration, inhalation, intracavernosal injection, intrathecal injection, epidural injection, or rectal administration, among others. For oral administration, for example, the pharmaceutical composition may be formulated as a tablet or may be coated or formulated to protect the active ingredient from degradation in the stomach. In addition, the composition may be administered by any device that allows the active substance to be delivered to target cells. The route of administration may vary depending on the general condition and age of the subject, the nature of the condition to be treated, and the choice of active ingredient.

In the pharmaceutical composition according to the present invention, the composition may be carried on a delivery vehicle. The delivery vehicle may include, but is not limited to, one or more selected from the group consisting of viral particles, vesicles, nanoparticles, microparticles, liposomes, transposons, micelles, antibodies, and exosomes.

In the pharmaceutical composition according to the present invention, the suitable dosage of the pharmaceutical composition may vary depending on factors such as the method of formulation, mode of administration, age, body weight, sex, pathological condition, food intake, time of administration, route of administration, rate of excretion, and responsiveness of the patient. A person skilled in the art can readily determine and prescribe an effective dose for the desired therapeutic or preventive effect. For example, the pharmaceutical composition may be administered as a single dose or in multiple doses, and may be administered one to four times per day. In one example, the pharmaceutical composition may be administered in an amount of 0.01 mg/kg to 100 mg/kg, preferably 0.02 mg/kg to 90 mg/kg, and more preferably 0.03 mg/kg to 80 mg/kg, based on an adult human subject.

In the pharmaceutical composition according to the present invention, the anticancer agent may be prepared in a unit dosage form or contained within a multi-dose container by formulation using pharmaceutically acceptable carriers and/or excipients according to methods easily implementable by those skilled in the art. The formulation may be in the form of a solution, suspension, or emulsion in an oil or aqueous medium, or in the form of an extract, powder, granule, tablet, or capsule, and may further include a dispersing agent or stabilizer. In addition, the pharmaceutical composition may be administered in the form of a suppository, spray, ointment, cream, gel, inhalant, or transdermal patch. Furthermore, the pharmaceutical composition may be formulated for administration to mammals, and more preferably, for administration to humans.

In the pharmaceutical composition according to the present invention, the pharmaceutically acceptable carrier may be solid or liquid, and may include one or more selected from the group consisting of excipients, antioxidants, buffers, bacteriostatic agents, dispersants, adsorbents, surfactants, binders, preservatives, disintegrants, sweeteners, flavoring agents, glidants, release controllers, humectants, stabilizers, suspending agents, and lubricants. Additionally, the pharmaceutically acceptable carrier may be selected from saline, sterile water, Ringer's solution, buffered saline, dextrose solution, maltodextrin solution, glycerol, ethanol, or mixtures thereof.

In one embodiment, a suitable filler may include sugars (e.g., dextrose, sucrose, maltose, and lactose), starches (e.g., corn starch), sugar alcohols (e.g., mannitol, sorbitol, maltitol, erythritol, and xylitol), starch hydrolysates (e.g., dextrin and maltodextrin), cellulose or cellulose derivatives (e.g., microcrystalline cellulose), or mixtures thereof, but is not limited thereto.

In one embodiment, a suitable binder may include povidone, copovidone, methylcellulose, hydroxyethylcellulose, hydroxypropylmethylcellulose, hydroxypropylcellulose, gelatin, gums, sucrose, starch, or mixtures thereof, but is not limited thereto.

In one embodiment, a suitable preservative may include benzoic acid, sodium benzoate, benzyl alcohol, butylated hydroxyanisole, butylated hydroxytoluene, chlorobutanol, gallates, hydroxybenzoates, EDTA, or mixtures thereof, but is not limited thereto.

In one embodiment, a suitable disintegrant may include sodium starch glycolate, cross-linked polyvinylpyrrolidone, cross-linked carboxymethylcellulose, starch, microcrystalline cellulose, or mixtures thereof, but is not limited thereto.

In one embodiment, a suitable sweetener may include sucralose, saccharin, sodium, potassium, or calcium saccharin, acesulfame potassium, sodium cyclamate, mannitol, fructose, sucrose, maltose, or mixtures thereof, but is not limited thereto.

In one embodiment, a suitable glidant may include silica, colloidal silicon dioxide, or talc, but is not limited thereto.

In one embodiment, a suitable lubricant may include long-chain fatty acids and their salts, such as magnesium stearate and stearic acid, talc, glyceryl wax, or mixtures thereof, but is not limited thereto.

### (2) Cosmetic Composition

According to the present invention, the anticancer agent may be used as a cosmetic composition for preventing or improving cancer.

In the cosmetic composition according to the present invention, the cosmetic composition may further comprise a dermatologically acceptable carrier. The dermatologically acceptable carrier may include purified water, oil, wax, fatty acid, fatty alcohol, fatty acid ester, surfactant, humectant, thickener, antioxidant, viscosity stabilizer, chelating agent, buffer, preservative, and lower alcohol, but is not limited thereto. The type and concentration thereof may vary and can be modified by those skilled in the art within the scope of the present invention.

In the cosmetic composition according to the present invention, the cosmetic composition may further comprise, in addition to the active ingredient of the present invention, functional substances such as whitening agents, moisturizers, anti-inflammatory agents, antibacterial agents, antifungal agents, vitamins, ultraviolet (UV) blockers, antibiotics, anti-acne agents, perfumes, or dyes, which can be included in the cosmetic composition of the present invention in amounts conventionally used in the cosmetic field. To enhance its functional effects, the cosmetic composition of the present invention may further contain one or more moisturizing active ingredients exhibiting the same or similar function as the composition of the present invention.

In the cosmetic composition according to the present invention, the cosmetic composition may be prepared in the form of a conventional emulsion formulation or solubilized formulation. Examples of cosmetics in the form of an emulsion formulation include nourishing lotions, creams, and essences, and an example of a cosmetic in the form of a solubilized formulation includes softening lotion. The cosmetic composition may be prepared in the form of a topically or systemically applicable adjunct conventionally used in the art by further comprising a dermatologically acceptable carrier or vehicle in addition to the active ingredient of the present invention. Suitable cosmetic formulations include, for example, a solution, gel, solid or paste anhydrous product, an emulsion obtained by dispersing an oil phase in an aqueous phase, suspension, microemulsion, microcapsule, microparticulate form, ionic (liposomal) or non-ionic vesicular dispersions, cream, skin lotion, powder, ointment, spray, or conceal stick. In addition, the cosmetic composition may also be prepared in the form of a foam or an aerosol composition further comprising a compressed propellant.

In the cosmetic composition according to the present invention, the cosmetic composition may be formulated in one or more forms selected from the group consisting of a solution, topical ointment, cream, foam, nourishing lotion, softening lotion, perfume, pack, softening water, emulsion, makeup base, essence, soap, liquid cleanser, bath additive, sunscreen cream, sun oil, suspension, emulsion, paste, gel, lotion, powder, soap, surfactant-containing cleanser, oil, powder foundation, emulsion foundation, wax foundation, patch, and spray.

### (3) Food Composition

As used herein, the term "food" refers to a natural product or processed product containing one or more nutrients, and preferably refers to a form that has undergone a certain degree of processing and is in a state suitable for direct consumption. In its conventional meaning, the term "food" may encompass foods, food additives, functional foods, and beverages.

As used herein, the terms "functional food" or "health-functional food" refer to a group of foods or food compositions to which added value has been imparted by physical, biochemical, biotechnological, or other methods, so that the functions of the respective food act or are expressed for a specific purpose. Such foods are designed and processed to sufficiently exhibit in vivo regulatory functions related to, for example, biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods. The types of the health supplements are not limited thereto, but may include, for example, powder, granule, tablet, capsule, or beverage forms.

According to the present invention, the anticancer agent may be used as a food composition for preventing or improving cancer.

In the food composition according to the present invention, the food may be selected from the group consisting of meat, sausage, bread, chocolate, candy, snacks, confectioneries, pizza, ramen, other noodles, gum, dairy products including ice cream, various soups, beverages, tea, coffee drinks, stamina drinks, alcoholic beverages, or vitamin complexes.

In the food composition according to the present invention, the food composition may further comprise various nutritional supplements, vitamins, electrolytes, flavoring agents, coloring agents, pectic acid and salts thereof, alginic acid and salts thereof, organic acids, protective colloid thickeners, pH adjusters, stabilizers, preservatives, glycerin, alcohol, carbonating agents used in carbonated beverages, and the like. In addition, the composition of the present invention may further comprise pulp for preparing natural fruit juices, fruit juice beverages, or vegetable beverages. These ingredients may be used independently or in combination.

In the food composition according to the present invention, the term "functional food" or "health-functional food" refers to a group of foods or food compositions to which added value has been imparted by physical, biochemical, biotechnological, or other methods, such that the functions of the respective food act or are expressed for a specific purpose. Such foods are designed and processed to sufficiently exhibit in vivo regulatory functions related to biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods.

### (4) Feed Composition

According to the present invention, the anticancer agent may be used as a feed composition for preventing or improving cancer.

In the feed composition according to the present invention, the feed comprises nutrients required by animals, such as energy, protein, lipids, vitamins, and minerals, and may be plant-based feed including grains, root crops, food processing by-products, algae, fibers, oils, starches, seed meals, and grain by-products, or animal-based feed including proteins, minerals, oils, mineral substances, and single-cell proteins, but is not limited thereto.

In the feed composition according to the present invention, the feed may be in the form of powdered feed, solid feed, moist pellet feed, dry pellet feed, extruder pellet (EP) feed, or raw feed, but is not limited thereto.

In the feed composition according to the present invention, the feed composition may further comprise binders, emulsifiers, preservatives, or the like, which are added to prevent quality deterioration, and may further comprise feed additives. To enhance efficacy, the feed may additionally comprise amino acids, vitamins, enzymes, flavoring agents, non-protein nitrogen compounds, silicates, buffers, extracts, oligosaccharides, and the like. The feed composition may further comprise feed premixes or other additives, but is not limited thereto.

### 2. Method for Prevention or Treatment of Cancer

The present invention provides a method for preventing or treating cancer, comprising administering to a subject a pharmaceutical composition comprising 1,1-diethoxyethane (1,1-DEE) as an active ingredient.

In the method according to the present invention, the subject may be a human, cow, monkey, horse, sheep, pig, chicken, turkey, quail, cat, dog, mouse, rat, rabbit, or guinea pig, but is not limited thereto.

In the method according to the present invention, the route, dosage, and frequency of administration of the pharmaceutical composition may be varied according to the condition of the patient and the presence or absence of side effects, and the optimal route, dosage, and frequency of administration can be selected by a person of ordinary skill in the art within an appropriate range. In the present invention, the preferred dosage of the pharmaceutical composition may be in the range of 0.001 mg/kg to 100 mg/kg per day for an adult, depending on the condition, body weight, sex, age, severity of the patient, and route of administration. The administration may be performed once per day or divided into multiple administrations. Such dosage ranges should not be construed as limiting the scope of the present invention in any respect.

The present invention relates, *inter alia,* to the provision of an anticancer agent comprising 1,1-diethoxyethane (1,1-DEE). The present invention also provides a pharmaceutical composition comprising 1,1,-DEE for use as an anticancer agent. Accordingly, it is understood that the present invention relates to the provision of 1,1-DEE or a composition comprising 1,1-DEE for use in the treatment of cancer.

According to the present invention and as shown in the appended non-limiting Examples, 1,1-DEE induces the generation of reactive oxygen species (ROS) in cancer cells.

The appended non-limiting Examples further show that the pharmaceutical composition according to the present invention comprising 1,1-DEE
i) induces apoptosis in cancer cells;
ii) induces cell cycle arrest by depleting G2/M phase cells and accumulating G1 phase cells; and/or
iii) decreases the expression of CDK3, CDK4, and cyclin D1, D3, and E proteins in cancer cells

Accordingly, the present invention relates to the following pharmaceutical/medical use items:
1. A pharmaceutical composition comprising 1,1-diethoxyethane (1,1,-DEE) for use in the treatment of cancer.
2. The pharmaceutical composition for use according to item 1, wherein said cancer is selected from the group consisting of selected from the group consisting of squamous cell carcinoma, basal cell carcinoma, melanoma, tumors of epithelial lining of glands or ducts, adenocarcinoma, papillary carcinoma, papillary adenocarcinoma of the liver and gallbladder ducts, hepatocellular carcinoma of the gastrointestinal tract, esophageal squamous cell carcinoma, esophageal adenocarcinoma, colorectal cancer, gastric cancer, airway tumors, bronchogenic carcinoma, small cell carcinoma, urinary tract transitional cell carcinoma, bladder squamous cell carcinoma, prostate cancer, cervical cancer, leukemia of hematopoietic cells and related cells, acute and chronic lymphocytic leukemia, polycythemia vera, lymphoid tissue cancers, Hodgkin lymphoma and non-Hodgkin lymphoma including malignant lymphomas, follicular lymphoma, diffuse lymphoma, small lymphocytic lymphoma, large cell lymphoma, lymphoblastic lymphoma, multiple myeloma, connective tissue tumors, osteosarcoma, nervous system tumors, neuroblastoma, retinoblastoma, glioblastoma, tumorigenic virus-associated glioma, Burkitt lymphoma, B cell lymphoma in immunocompetent subjects, nasopharyngeal carcinoma, esophageal and gastroesophageal cancer, squamous cell carcinoma, pancreatic islet tumors, breast cancer, lung cancer, colorectal cancer, retinoblastoma, liver cancer, pancreatic cancer, brain cancer, malignant mesothelioma, hepatitis B virus-related hepatocellular carcinoma, endometrial cancer, ovarian cancer, head and neck cancer, thyroid cancer, and soft tissue-related cancers.
3. The pharmaceutical composition for use according to item 1, wherein said cancer is leukemia.
4. The pharmaceutical composition for use according to item 3, wherein said leukemia is is selected from the group consisting of acute lymphoblastic leukemia (ALL), acute lymphoblastic B-cell leukemia, acute lymphoblastic T-cell leukemia, acute myeloblastic leukemia (AML), acute promyelocytic leukemia (APL), acute monocytic leukemia, acute erythroid leukemia, acute megakaryoblastic leukemia, acute myelomonocytic leukemia, acute undifferentiated leukemia, chronic myeloid leukemia, chronic lymphocytic leukemia, T-cell precursor lymphoblastic leukemia, T-cell prolymphocytic leukemia, Philadelphia chromosome-positive leukemia, FLT3 mutation-positive acute myeloblastic leukemia, and acute erythroid leukemia.
   As shown in the appended, non-limiting examples, the cancer may be a hematological cancer, particularly a leukemia. For example, the leukemia may be an Acute Lymphoblastic Leukemia (ALL), such as T-cell ALL (T-ALL). Accordingly, the anticancer agent according to the present invention (i.e. 1,1-DEE) or the pharmaceutical composition comprising 1,1-DEE may be used in the treatment of cancer. For example, 1,1-DEE or the pharmaceutical composition comprising 1,1-DEE may be used in the treatment of a hematological cancer such as a leukemia. In a particular embodiment of the invention, 1,1-DEE or the pharmaceutical composition comprising 1,1-DEE may be used in the treatment of ALL, such as T-ALL.
5. The pharmaceutical composition for use according to any one of items 1 to 4, wherein the composition elicits anticancer activity by:
   i) inducing apoptosis in cancer cells;
   ii) inducing cell cycle arrest by depleting G2/M phase cells and accumulating G1 phase cells; and/or
   iii) decreasing the expression of CDK3, CDK4, and cyclin D1, D3, and E proteins in cancer cells.

The skilled artisan is aware of means and methods to measure/determine features such as apoptosis, cell cycle state, cell cycle phase, depletion of cells in certain cell cycle phases such as G2/M phases (i.e. depletion of G2/M phase cells), accumulation of cells in certain cell cycle phases such as G1 phase (i.e. accumulation of G1 phase cells), expression of CDK3, CDK4, and cyclin D1, D3, and E proteins in cells (e.g. cancer cells). Suitable techniques to assess these parameters are abundantly known in the art. Experimental guidance is, *inter alia* provided in the appended Examples.

It is understood that cell cycle arrest refers to interrupting cell cycle progression e.g. of a cancer cell, thereby leading to the accumulation of cells in a specific cell cycle phase. In one example, this cell cycle phase may be the G1 phase.

1,1-DEE or the pharmaceutical composition comprising 1,1-DEE restores and/or further restores vitality during the course of cancer treatment.

The pharmaceutical composition for use according to item 5, wherein said composition comprises 1,1-DEE.

1,1-DEE or the pharmaceutical composition comprising 1,1-DEE may be administered alone, but also in combination with an additional cancer therapy. Accordingly, the present invention relates to the provision of 1,1-DEE or a pharmaceutical composition comprising 1,1,-DEE for use in the treatment of cancer in combination with an additional cancer therapy. Such additional cancer therapy may, for example, be an immunotherapeutic agent, a chemotherapeutic agent, a radioprotective agent, a radiotherapy, or a gene therapy.

For example, 1,1-DEE or the pharmaceutical composition comprising 1,1-DEE are for use in combination with an immune checkpoint inhibitor, such as an antibody directed against PD-1, PD-L1 and/or CTLA-4, a cytokine, a CAR-T cell, an oncolytic virus, and a cancer vaccine.

Alternatively or additionally, 1,1-DEE or the pharmaceutical composition comprising 1,1-DEE are for use in combination with chemotherapeutic agent such as an antimetabolite, a platinum-based chemotherapeutic compound, an alkylating agent, and a topoisomerase inhibitor.

Alternatively or additionally, 1,1-DEE or the pharmaceutical composition comprising 1,1-DEE are for use in combination with radiotherapy, such as external beam radiation, brachytherapy, proton therapy, radiofrequency ablation, stereotactic radiosurgery, or neutron therapy.

As further items, the present invention also provides for food, feed and cosmetic compositions comprising the anti-cancer agent (i.e. 1,1-DEE) according to the present invention. Such compositions may be generated by adding the anti-cancer agent according to the present invention (i.e. 1,1-DEE) to the respective composition.

Hereinafter, various examples are provided to facilitate understanding of the invention. The following examples are provided solely to aid in the understanding of the invention and are not intended to limit the scope of the invention to these examples.

### <Materials and Methods>

### 1. Cell Culture

Jurkat E6-1 (KCLB No: 40152) cells were purchased from the Korean Cell Line Bank (Seoul, Jongno-gu, Daehak-ro, Republic of Korea) and cultured in RPMI 1640 medium supplemented with 10% fetal bovine serum (FBS) and 1% penicillin-streptomycin at 37°C in a humidified incubator containing 5% CO₂.

### 2. Cell Viability Assay

### Cells were seeded in a 96-well plate at a density of 2

× 10⁴ cells per well in 100 µL of RPMI 1640 medium supplemented with 10% FBS and 1% penicillin-streptomycin. The following day, 1,1-diethoxyethane (1,1-DEE; Sigma-Aldrich, St. Louis, USA) and 1,2-diethoxyethane (1,2-DEE; TCI, Tokyo Chemical Industry Co., Ltd., Japan) were serially diluted and added to each well for 24 hours (total volume: 120 µL per well). Thereafter, 10 µL of MTT solution (DoGenBio Co., Ltd.) was added to each well, and the plate was incubated at 37°C for 2 hours. Cell viability was determined by measuring absorbance at 450 nm using a microplate spectrophotometer (Epoch, Biotek, USA).

### 3. Western Blot Analysis

To extract proteins from Jurkat cells (5 × 10⁵/mL) cultured in 60 × 15 mm cell culture dishes (SPL Life Sciences, Gyeonggi-do, Republic of Korea), 150 µL of Pro-PREP^{™} protein extraction solution (iNtRON Biotechnology, Gyeonggi-do, South Korea) was added per dish. Polyvinylidene fluoride (PVDF) membranes and Western chemiluminescent HRP substrate were obtained from Millipore Corporation (Billerica, MA, USA). A total of 30 µg of protein was separated by 10% SDS-polyacrylamide gel electrophoresis (SDS-PAGE) and subsequently transferred onto PVDF membranes for further analysis. The membranes were blocked in TBST containing 5% defatted milk with 0.1% Tween-20 for 1-2 hours and incubated overnight at 4°C with primary antibodies (1:1000) diluted in TBST. After washing three times for 10 minutes each with TBST, membranes were incubated with horseradish peroxidase (HRP)-conjugated secondary antibodies (1:2000) to detect immunoreactive proteins via chemiluminescence. The following antibodies were used in the Western blot experiments: anti-phospho-AMPK (#2535S), anti-AMPK (#2532S), anti-Bax (2772S), anti-Cyclin D1 (#29226S), anti-Cyclin D3 (#2936S), anti-HK II (B-8, SC374091), anti-LDHA (E-9, SC137243), anti-Bcl2 (SC509), anti-Cyclin E (SC377100), anti-Caspase3 (SC373730), anti-Caspase9 (SC56073), anti-CDK2 (SC-6248), anti-CDK4 (SC-23896), Total OXPHOS Cocktail (Abcam, 110411), and anti-β-actin (5125S) monoclonal antibodies (Cell Signaling Technology, Danvers, MA, USA). To determine total protein levels, the blotted membranes were washed in Restore^{™} Western Blot Stripping Buffer (Thermo Fisher Scientific, Rockford, IL, USA) at 56°C for 30 minutes.

### 4. Cell Apoptosis Assay - Annexin V/FITC Staining

To evaluate the apoptotic potential of Jurkat cells, an Annexin V fluorescence kit (BD Pharmingen, San Diego, USA) was used according to the manufacturer's protocol. Jurkat cells were seeded in 6-well plates at a density of 2.0 × 10 ⁵ cells per well in RPMI medium supplemented with 10% FBS and incubated for 24 hours. After seeding, cells were treated with the indicated concentrations of 1,1-DEE and incubated at 37°C for 24 hours. Following treatment, cells were harvested, pelleted by centrifugation, and resuspended in 400 µL of binding buffer. Cells were stained with 5 µL of FITC-Annexin V and 10 µL of propidium iodide (PI) provided in the kit. Apoptosis was analyzed using flow cytometry (BD FACSCalibur, BD Biosciences, CA, USA), and data plotting and analysis were performed with FlowJo^{™} Software (BD Biosciences, CA, USA).

### 5. Cell Cycle Analysis

Jurkat cells were seeded in 6-well plates at a density of 5 × 10⁵ cells/mL and cultured at 37°C for 24 hours. Cells were then treated with the desired concentration of 1,1-DEE for 24 hours. Following treatment, cells were detached using trypsin-EDTA, centrifuged, and washed with 1X cold PBS, then fixed in 70% ethanol at 4°C for 30 minutes. For analysis, cells were washed with 1X PBS and centrifuged at 1,200 rpm for 5 minutes. Cells were subsequently resuspended in 500 µL of solution containing 10 µg/mL RNase A (Sigma-Aldrich) and 75 µM propidium iodide (Sigma-Aldrich) and incubated at room temperature in the dark for 1 hour. DNA fluorescence intensity was measured using a flow cytometer equipped with a BD FACSVerse, and data were plotted and analyzed using FlowJo^{™} software.

### 6. Measurement of ROS Generation

Intracellular H₂O₂ levels were measured using 5- and 6-chloromethyl-2',7'-dichlorodihydrofluorescein diacetate (DCFDA; Molecular Probes, Eugene, OR, USA). Jurkat cells were cultured in RPMI medium supplemented with 10% FBS until reaching 80% confluence. To evaluate ROS generation induced by 1,1-DEE, cells were pretreated with 2 mM NAC, 250 µM Trolox, or 10 µM Ebselen 1 hour prior to compound treatment. Cells were then incubated with 10 µM DCFDA for 15 minutes and observed using a laser scanning confocal microscope (Carl Zeiss, Jena, Germany). DCFDA fluorescence was excited with an argon laser at 488 nm, and emission was collected using a 515 nm long-pass filter. Following incubation with DCFDA, ROS levels were quantified by flow cytometry using a BD FACSCalibur (BD Biosciences, Franklin Lakes, NJ, USA). Data were plotted and analyzed using FlowJo^{™} software version 10.10 (BD Biosciences, San Jose, CA, USA).

### 7. Animal Care and Drug Administration

Four-week-old immunodeficient male NSG mice (NOD.Cg-Prkdc^scid^IL2rγ^tm1Wjl^/Szj) were purchased from Jackson Laboratory (Bar Harbor, ME, USA) and maintained at the Center for Laboratory Animal Resources, Chonnam National University (CNU IACUC-H-2024-42). Mice were housed under a 16 h light/8 h dark cycle at 23°C and 60% humidity, with ad libitum access to food and water. Five mice were housed per cage, and body weights were recorded three times per week. All experiments were conducted in accordance with institutional guidelines of Chonnam National University. Jurkat cells cultured in RPMI medium supplemented with 10% FBS were suspended in 100 µL of PBS at a density of 2 × 10⁷ cells per mouse and injected subcutaneously into the right flank of each mouse. Mice were divided into control and treatment groups. Four weeks after tumor induction, the treatment group received intratumoral injections of 1,1-DEE (110 mg/kg) every other day from day 2 to day 16, whereas the control group received PBS. Eighteen days after drug administration, animals were used for survival studies. Additionally, tumor tissues and major organs were collected for histological analysis by hematoxylin and eosin (H&E) staining.

### 8. Enzyme-Linked Immunosorbent Assay (ELISA)

For in vivo studies, plasma was collected from blood obtained via cardiac puncture of anesthetized NSG mice. The levels of cytokine IL-8 were quantified using an ELISA kit (R&D Systems, USA). Optical density measurements were performed with a microplate spectrophotometer (Epoch, BioTek Instruments, USA) and a microplate strip washer (ELx50, BioTek Instruments, USA). IL-8 concentrations were calculated based on a standard curve and expressed in pg/mL.

### 9. Lactate Production Assay

Jurkat cells were seeded in 96-well plates at a density of 1 × 10⁴ cells per well (100 µL). Once the cells reached confluence, they were treated with 1,1-DEE at 1, 2, and 5 mM or 1,2-DEE at 5 mM for 2 hours. Thereafter, culture media and cells were separately collected, and lactate production was measured using the Lactate-Glo^{™} Assay (Promega, USA) according to the manufacturer's instructions. Luminescence was detected using a GloMax plate reader (Promega, USA).

### 10. Glucose Uptake Assay

Jurkat cells were seeded in 96-well plates at a density of 1 × 10⁴ cells per well (100 µL). Once the cells reached confluence, they were treated with 1,1-DEE at 1, 2, and 5 mM or 1,2-DEE at 5 mM for 2 hours. Glucose uptake was assessed using the Glucose Uptake-Glo^{™} Assay (Promega, USA) according to the manufacturer's instructions. Luminescence was recorded using a GloMax^{®} luminometer (Promega, USA) with an integration time of 0.5 seconds, following the Glucose Uptake-Glo^{™} protocol.

### 11. Polymerase Chain Reaction (RT-PCR)

Total RNA was extracted from HT-29 cells using TRIzol reagent (Invitrogen, Carlsbad, USA). First-strand complementary DNA (cDNA) was synthesized from 1 µg of total RNA using random primers and M-MLV reverse transcriptase (Promega, USA). PCR amplification of cDNA for β-actin and IL-6 was performed using primer sets and PCR master mix solution (iNtRON, South Korea). The primers used were as follows:
β-actin forward: 5'-AAG CAG GAG TAT GAC GAG TC-3'
β-actin reverse: 5'-GCC TTC ATA CAT CTC AAG TT-3' (561 bp)
LDHA forward: 5'-CAC CAT GAT TAA GGG TCA TTA C-3'
LDHA reverse: 5'-AGG TCA GAG ATT CCA TTC TG-3' (87 bp)
HK2 forward: 5'-GAG CCA CCA CTC ACC CTA CT-3'
HK2 reverse: 5'-CCA GGC ATT CGG CAA TGT G-3' (249 bp)
GLUT1 forward: 5'-AGT TCT ACA ACC AGA CAT GG-3'
GLUT1 reverse: 5'-CAG GTT CAT CAT CAG CAT TG-3' (179 bp)
PCR was performed under the following conditions: denaturation at 94°C for 30 seconds, annealing at 54°C for 20 seconds, and extension at 72°C for 30 seconds.

### 12. Histological Analysis (H&E Staining)

After measuring tumor volume and weight, the animals were sacrificed for histological studies. The tissues were dehydrated twice in 95% ethanol for 0.5 hours each, immersed in xylene at 60-70°C for 1 hour, and embedded in paraffin for 12 hours. Tissue cryosections with a thickness of 6 µm were prepared using FSC 22 Clear (Leica) for fixation. The sections were stained with Harris' hematoxylin solution at 60-70°C for 6 hours and rinsed with tap water until colorless. Subsequently, the sections were treated twice with a mixture of 10% acetic acid and 85% ethanol for 2 hours and 10 hours, respectively, and rinsed with tap water for differentiation. For bluing, the sections were immersed in a saturated lithium carbonate solution for 12 hours and rinsed again with tap water. Finally, counterstaining was performed with eosin Y ethanol solution for 48 hours.

### 13. Measurement of Mitochondrial Membrane Potential (JC-1 Staining, Flow Cytometry)

Mitochondrial membrane potential was assessed using JC-1 dye (Beyotime, Seoul, Korea) . Jurkat cells were cultured for 24 hours and treated with the indicated concentrations of the compound, followed by incubation with JC-1 (20 nM) at 37°C in the dark for 30 minutes. After washing with PBS, cells were either plated on confocal dishes (Carl Zeiss, Germany) for imaging or trypsinized, collected, and analyzed using a BD FACS Calibur flow cytometer (BD Biosciences, USA) . Data analysis was performed using FlowJo^{™} software (BD Biosciences, USA).

### 14. Statistical Analysis

All data represent the mean ± standard deviation (SD) of three independent experiments. GraphPad Prism software (Version 8.0) was used for data presentation. Multivariable analyses were performed using one-way ANOVA with Tukey's multiple comparison test. Statistical significance was considered at p < 0.05 (#, *, ^, +, @), p < 0.01 (##, **, ^^, ++, @@), p < 0.001 (###, ***, ^^^, +++, @@@), and p < 0.0001 (####, ****, ^^^^, ++++, @@@@). Tumor volume and weight were analyzed using Student's t-test, while survival rates were compared using the Log-Rank test. The significance level for all tests was set at p < 0.05.

### <Results>

### Experimental example 1. Evaluation of the anti-proliferative and apoptosis-inducing effects of 1,1-DEE in ALL cell lines

To assess the anti-proliferative and apoptosis-inducing effects of 1,1-diethoxyethane (1,1-DEE), Jurkat E6.1 cells were used. MTT assays demonstrated that 1,1-DEE reduced the viability of Jurkat E6.1 cells in a concentration-dependent manner, even at concentrations below 10 mM (Figures 1A and 1B). In contrast, 1,2-DEE, used as a negative control, did not produce significant changes in cell viability (Figures 1C and 1D).

To evaluate the cytotoxicity of low-dose 1,1-DEE, Jurkat E6.2 cells were treated with various concentrations of 1,1-DEE for 6, 12, and 24 hours. The cells exhibited a rounded and shrunken morphology, which became more pronounced with increasing concentration and treatment duration (Figure 1E). No morphological changes were observed in the 1,2-DEE-treated group (Figure 1E). Notably, approximately a 2-fold change was observed after 6 and 12 hours of treatment, while a strong inhibitory effect (~3-fold) was observed at 24 hours.

To determine the induction of apoptosis, Jurkat E6.1 cells were treated with 1,1-DEE for 24 hours and subsequently stained with Annexin V and PI (Figure 2A). Analysis revealed a significant increase in apoptotic cells upon 1,1-DEE treatment, with the proportion of cells in quadrant Q2 increasing in a concentration-dependent manner (Figure 2B).

Western blot analysis further demonstrated that 1,1-DEE treatment increased the expression of the pro-apoptotic protein Bax, while the expression of the anti-apoptotic protein Bcl-2 and caspase-9 and caspase-3 was decreased. In contrast, no significant changes were observed in cells treated with the positive control Compound C (10 µM, AMPK inhibitor) or 1,2-DEE compared to the untreated control (Figures 2C and 2D).

Collectively, these results indicate that 1,1-DEE induces apoptosis in Jurkat E6.1 cells, leading to inhibition of cell proliferation.

### Experimental example 2. Evaluation of cell cycle arrest via ROS generation induced by 1,1-DEE in ALL cell lines

Increased intracellular levels of reactive oxygen species (ROS) are known to induce oxidative stress, leading to cell cycle arrest and apoptosis in cancer cells. ROS can disrupt the intracellular redox balance and upregulate the expression of CDK inhibitor proteins such as p21 and p27, thereby inducing cell cycle arrest at the G0/G1 or G2/M phases. Accordingly, this study aimed to evaluate whether 1,1-diethoxyethane (1,1-DEE) induces ROS generation and cell cycle arrest, thereby suppressing tumor growth.

First, Jurkat E6.1 cells were used to analyze cell cycle alterations following 1,1-DEE treatment. Cells were treated with 1-10 mM 1,1-DEE for 24 hours, and cell cycle distribution was analyzed by flow cytometry. The results revealed that 1,1-DEE treatment decreased the proportion of cells in the G2/M phase while leading to accumulation of cells in the G1 phase (Figures 3A and 3B).

Western blot analysis demonstrated that 1,1-DEE reduced the expression of CDK2, CDK4, cyclin D1, cyclin D3, and cyclin E proteins. In contrast, the positive control cytochrome c (10 µM) or 1,2-DEE-treated groups showed no significant changes compared to untreated controls (Figures 3C and 3D). Notably, even the lowest concentration of 1 mM 1,1-DEE induced significant accumulation of G1-phase cells, indicating that 1,1-DEE induces cell cycle arrest in a concentration-dependent manner.

ROS accumulation is known to play a critical role in the regulation of cell proliferation and apoptosis. Therefore, the extent of ROS generation following 1,1-DEE treatment was evaluated using the fluorescent probe DCFH-DA. Treatment with 1,1-DEE (5 mM) induced a marked increase in ROS levels within 30-60 minutes (Figure 9).

To determine whether 1,1-DEE-induced ROS accumulation contributes to growth inhibition, cells were treated with 1,1-DEE in the presence of ROS scavengers (NAC, Trolox, Ebselen) or a ROS inducer (H₂O₂), and cell viability was assessed (Figure 4A). Under conditions without NAC, 1,1-DEE induced ROS accumulation and growth inhibition. In contrast, in the presence of NAC, ROS accumulation induced by 1,1-DEE was reduced, resulting in increased cell viability (Figures 4C and 4D).

These results indicate that ROS generation plays a critical role in the cell growth inhibitory effects of 1,1-DEE.

### Experimental Example 3. Evaluation of Warburg Effect Suppression via AMPK Signaling Modulation by 1,1-DEE in ALL Cells

AMP-activated protein kinase (AMPK) is a conserved serine/threonine kinase that serves as a central regulator of intracellular energy homeostasis. Activation of AMPK is known to modulate key signaling pathways that drive metabolic reprogramming in tumor cells, thereby altering their bioenergetic properties. Saito et al. reported that AMPK plays a critical role in maintaining the survival of leukemia cells in the bone marrow by inhibiting glucose transporter (GLUT)-mediated glucose uptake and promoting ROS accumulation. Conversely, genetic loss of AMPK impairs glucose utilization, rendering leukemia cells more sensitive to metabolic stress.

In this study, Jurkat E6.1 cells were used to evaluate whether 1,1-DEE activates AMPK. As shown in Figures 5A and 5B, 1,1-DEE induced time-dependent activation of AMPK, with a significant increase in AMPK activity observed at 60 minutes post-treatment. Furthermore, 1,1-DEE induced phosphorylation of AMPK in a concentration-dependent manner (Figures 5C and 5D), with the most pronounced activation observed at 5 mM.

Tumor cells are known to undergo metabolic reprogramming to rewire nutrient utilization pathways in order to meet energy demands for growth. Notably, cancer cells often exhibit the Warburg effect, converting glucose to lactate even in the presence of sufficient oxygen. Accordingly, this study evaluated whether 1,1-DEE-induced AMPK activation can suppress the Warburg effect.

Treatment with 1,1-DEE at concentrations of 1, 2, and 5 mM significantly reduced glucose uptake in Jurkat E6.1 cells concomitant with AMPK activation (p < 0.05) (Figure 6A). Even the lowest concentration of 1 mM showed a clear inhibitory effect, indicating that 1,1-DEE exerts a potent and concentration-dependent suppression of glycolytic activity. Additionally, treatment with 1,1-DEE at 2 and 5 mM markedly decreased lactate production, suggesting inhibition of glycolytic flux (Figure 6B).

These results demonstrate that 1,1-DEE suppresses the Warburg effect by promoting AMPK phosphorylation and that upregulation of AMPK alone is sufficient to reduce glycolytic activity in the cells.

### Experimental Example 4. Evaluation of AMPK Regulation, Mitochondrial Membrane Potential, and Glycolytic Properties by 1,1-DEE in ALL Cells

Aerobic glycolysis in cancer cells primarily converts pyruvate to lactate rather than directing it into the mitochondrial TCA cycle, thereby reducing oxidative phosphorylation (OXPHOS) activity. This metabolic shift limits ROS production within mitochondria, maintaining mitochondrial structural integrity, while moderate ROS levels support tumor progression and survival. Therefore, targeting glucose uptake, rate-limiting glycolytic enzymes, and mitochondrial OXPHOS is considered a strategic approach to suppress the Warburg effect and block metabolic reprogramming in tumor cells.

In this study, we investigated whether 1,1-DEE exerts anti-Warburg effects by modulating the expression and activity of glycolysis-related factors.

To evaluate the effect of 1,1-DEE on mitochondrial OXPHOS activity, Jurkat E6.1 cells were treated with 2 mM 1,1-DEE, and Western blot analysis was performed using an OXPHOS antibody cocktail containing antibodies against mitochondrially encoded cytochrome c oxidase subunit 2 (CO2) and four nuclear-encoded polypeptides (NDUFB8, SDHB, UQCRC2, ATP5A) (Figures 6C-6H). The results showed that expression of these complex-specific proteins increased in a time-dependent manner following 1,1-DEE treatment, indicating enhanced mitochondrial respiratory activity compared to untreated controls. These findings suggest that 1,1-DEE promotes mitochondrial OXPHOS, thereby enhancing cellular bioenergetic function.

To assess whether 1,1-DEE directly regulates the glucose transporter GLUT1, Jurkat E6.1 cells were treated with 2 mM 1,1-DEE for 4 hours, followed by analysis of GLUT1 protein levels. GLUT1 expression was significantly reduced (Figures 7I-7K), indicating that 1,1-DEE suppresses GLUT1 expression at both transcriptional and translational levels. Consequently, 1,1-DEE effectively inhibits GLUT1-mediated glycolytic activity.

Overexpression of LDHA (lactate dehydrogenase A) and HK2 (hexokinase 2) is closely associated with elevated lactate production, increased tumor invasiveness, and resistance to chemotherapeutic and radiotherapeutic treatments. In this study, treatment with 2 mM 1,1-DEE for 4 hours markedly reduced LDHA and HK2 protein levels compared to untreated controls, suggesting that 1,1-DEE effectively suppresses key regulators of glycolysis and inhibits aerobic glycolytic activity in cancer cells.

Mitochondrial membrane potential (ΔΨm) is an important indicator of cell viability and function. JC-1 staining was employed to evaluate ΔΨm. JC-1, a cationic cyanine dye, forms red fluorescent aggregates in polarized mitochondria, whereas depolarized mitochondria display green monomer fluorescence, enabling analysis of mitochondrial polarization or depolarization based on fluorescence.

Treatment of Jurkat E6.1 cells with 1,1-DEE resulted in a concentration-dependent increase in red fluorescence, indicating enhanced mitochondrial polarization. In contrast, 1,2-DEE treatment showed balanced red/green fluorescence with no significant changes in membrane potential. Notably, strong red fluorescence predominated in the 1,1-DEE-treated group (Figure 7), suggesting that 1,1-DEE supports or enhances mitochondrial membrane potential, thereby maintaining mitochondrial function.

### Experimental Example 5. Evaluation of In Vivo Anti-Tumor Effect of 1,1-DEE on ALL Cells

To further assess whether 1,1-DEE inhibits tumor growth in vivo, a subcutaneous xenograft model was established using 4-week-old NSG mice. Jurkat cells were stably injected subcutaneously, and tumor volume was measured four weeks post-injection. Subsequently, 1,1-DEE at the desired concentration was administered directly into the tumor site every two days for a total of eight injections, and changes in tumor volume were continuously monitored.

The results demonstrated that 1,1-DEE significantly suppressed tumor growth in vivo (Figure 8A). Comparison of tumor growth curves among the groups revealed that, 18 days post-cell injection, tumor volume was markedly reduced in the 1,1-DEE-treated group (Figures 8B, 8C). Body weight of the mice was monitored (Figure 8D), and tumor size and survival were compared between the saline-treated control and 1,1-DEE-treated groups at day 18. Kaplan-Meier survival analysis showed that the 1,1-DEE-treated group exhibited a significantly prolonged survival period compared to the untreated control (Figure 8E).

To evaluate potential organ toxicity 18 days after 1,1-DEE treatment, detailed histopathological analyses were performed. Pathological changes in major organs, including heart, liver, kidney, spleen, and lung, were assessed, and representative microscopic images are shown in Figure 8F.

In the livers of tumor-bearing mice, pronounced morphological alterations were observed compared to normal mice. While liver tissue in normal mice displayed regularly arranged central veins and portal veins, tumor-bearing mice exhibited lymphocyte infiltration and irregular central and portal vein structures. However, in tumor-bearing mice treated with 1,1-DEE, the central and portal vein structures were adequately maintained, and cellular infiltration was minimized, indicating partial restoration of the disrupted liver architecture.

Kidney tissue displayed acute vacuolation, expansion of epithelial linings, nuclear degeneration, necrosis, and epithelial alterations. Heart tissue exhibited chemodectoma, toxic myocarditis, reddish-brown atrophy, and yellowish-brown pigmentation, corresponding to lipofuscin granules, presumed remnants of cellular organelles and cytoplasmic material. In the lungs, vacuolation, central vein degeneration, inflammation, hemorrhage, altered cellular structures, hemosiderophages, and other lesions were observed.

These results indicate that 1,1-DEE treatment can restore liver tissue structure to a state similar to that of normal animals. Moreover, H&E staining of tumor-bearing tissues revealed that the heart tissue in the 1,1-DEE-treated group maintained normal tissue architecture, whereas the heart tissue in untreated tumor-bearing mice displayed irregular structural changes.

Recent studies have indicated that interleukin-8 (IL-8) serves as a key biomarker associated with disease progression and poor prognosis in various types of leukemia. Meanwhile, activation of AMP-activated protein kinase (AMPK) has been reported to suppress IL-8 expression through inhibition of NF-κB. In this study, we investigated whether 1,1-DEE could activate AMPK, thereby suppressing IL-8 expression and alleviating leukemia-associated inflammatory responses (Figure 10). ELISA analysis revealed that IL-8 expression was elevated in control mice, whereas IL-8 levels were significantly reduced in the 1,1-DEE-treated group.

In conclusion, this study demonstrates that 1,1-DEE suppresses the Warburg effect by increasing mitochondrial membrane potential (ΔΨm) and promoting OXPHOS activity, while simultaneously reducing the expression of key glycolytic enzymes, including LDHA and HK2, thereby inhibiting glycolytic flux. Through this time- and dose-dependent regulation of cellular metabolism, 1,1-DEE exhibits potent anticancer activity and may serve as a promising therapeutic candidate targeting metabolic pathways.

As described above, specific aspects of the present invention have been described in detail. It will be apparent to those skilled in the art that such specific descriptions are merely preferred embodiments, and the scope of the present invention is not limited thereby. Accordingly, the actual scope of the present invention is defined by the appended claims and their equivalents.

This invention was supported by a national research and development project.
[Project Number] 2018R1D1A1B06051438
[Ministry] Ministry of Science and ICT
[Project Management (Specialized) Organization] National Research Foundation of Korea
[Project Title] Alcohol-Induced Regulation of the Tumor Suppressor Protein PTEN
[Project Implementing Organization] Chonnam National University

## Claims

1. An anticancer agent comprising 1,1-diethoxyethane (1,1-DEE) as an active ingredient.

2. The anticancer agent according to claim 1, wherein the 1,1-DEE induces the generation of reactive oxygen species (ROS) in cancer cells.

3. The anticancer agent according to claim 1, wherein the 1,1-DEE induces apoptosis in cancer cells.

4. The anticancer agent according to claim 1, wherein the 1,1-DEE induces cell cycle arrest by depleting G2/M phase cells and accumulating G1 phase cells, and decreases the expression of CDK3, CDK4, and cyclin D1, D3, and E proteins in cancer cells.

5. The anticancer agent according to claim 1, wherein the agent is for use in treating a hematological malignancy or a solid tumor.

6. The anticancer agent according to claim 1, wherein the anticancer agent is for use in treating cancers selected from the group consisting of squamous cell carcinoma, basal cell carcinoma, melanoma, tumors of epithelial lining of glands or ducts, adenocarcinoma, papillary carcinoma, papillary adenocarcinoma of the liver and gallbladder ducts, hepatocellular carcinoma of the gastrointestinal tract, esophageal squamous cell carcinoma, esophageal adenocarcinoma, colorectal cancer, gastric cancer, airway tumors, bronchogenic carcinoma, small cell carcinoma, urinary tract transitional cell carcinoma, bladder squamous cell carcinoma, prostate cancer, cervical cancer, leukemia of hematopoietic cells and related cells, acute and chronic lymphocytic leukemia, polycythemia vera, lymphoid tissue cancers, Hodgkin lymphoma and non-Hodgkin lymphoma including malignant lymphomas, follicular lymphoma, diffuse lymphoma, small lymphocytic lymphoma, large cell lymphoma, lymphoblastic lymphoma, multiple myeloma, connective tissue tumors, osteosarcoma, nervous system tumors, neuroblastoma, retinoblastoma, glioblastoma, tumorigenic virus-associated glioma, Burkitt lymphoma, B cell lymphoma in immunocompetent subjects, nasopharyngeal carcinoma, esophageal and gastroesophageal cancer, squamous cell carcinoma, pancreatic islet tumors, breast cancer, lung cancer, colorectal cancer, retinoblastoma, liver cancer, pancreatic cancer, brain cancer, malignant mesothelioma, hepatitis B virus-related hepatocellular carcinoma, endometrial cancer, ovarian cancer, head and neck cancer, thyroid cancer, and soft tissue-related cancers.

7. The anticancer agent according to claim 1, when the anticancer agent is used for leukemia, the leukemia is selected from the group consisting of acute lymphoblastic leukemia (ALL), acute lymphoblastic B-cell leukemia, acute lymphoblastic T-cell leukemia, acute myeloblastic leukemia (AML), acute promyelocytic leukemia (APL), acute monocytic leukemia, acute erythroid leukemia, acute megakaryoblastic leukemia, acute myelomonocytic leukemia, acute undifferentiated leukemia, chronic myeloid leukemia, chronic lymphocytic leukemia, T-cell precursor lymphoblastic leukemia, T-cell prolymphocytic leukemia, Philadelphia chromosome-positive leukemia, FLT3 mutation-positive acute myeloblastic leukemia, and acute erythroid leukemia.

8. The anticancer agent according to claim 1, wherein the anticancer agent is administered in combination with one or more of an immunotherapeutic agent, monoclonal antibody, chemotherapeutic agent, radioprotective agent, radiotherapeutic agent, and gene therapy agent,
the immunotherapeutic agent includes immune checkpoint inhibitors comprising PD-1, PD-L1, and CTLA-4 inhibitors, cytokine therapy comprising interleukins and interferons, CAR-T cell therapy, oncolytic viruses, vaccine therapy, or targeted therapy,
the chemotherapeutic agent includes antimetabolites, platinum-based compounds, alkylating agents, or topoisomerase inhibitors, and
the radiotherapeutic agent includes external beam radiation, brachytherapy, proton therapy, radiofrequency ablation, stereotactic radiosurgery, or neutron therapy.

9. The anticancer agent according to claim 1, wherein the agent is to prevent the onset of cancer.

10. The anticancer agent according to claim 1, wherein the agent exhibits an effect of restoring vitality during the course of cancer treatment.

11. The anticancer agent according to claim 1, wherein the anticancer agent is used as a pharmaceutical composition for preventing or treating cancer.

12. The anticancer agent according to claim 11, wherein the pharmaceutical composition is administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, and rectal administration.

13. The anticancer agent according to claim 1, wherein the anticancer agent is used as a cosmetic composition for preventing or improving cancer.

14. The anticancer agent according to claim 13, wherein the cosmetic composition is formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, and spray.

15. The anticancer agent according to claim 1, wherein the anticancer agent is used as a food composition for preventing or improving cancer.

16. The anticancer agent according to claim 15, wherein the food includes meat, sausage, bread, chocolate, candy, snacks, cookies, pizza, ramen, other noodles, chewing gum, ice cream and dairy products, various soups, beverages, tea, coffee beverages, stamina drinks, alcoholic beverages, or vitamin complexes.

17. The anticancer agent according to claim 1, wherein the anticancer agent is used as a feed composition for preventing or improving cancer.

18. The anticancer agent according to claim 17, wherein the feed includes powdered feed, solid feed, moist pellet feed, dry pellet feed, extruder pellet (EP) feed, or raw feed.
